(19) Europäisches Patentamt

European Patent Office

Office européen des brevets

(11) **EP 1 466 980 B1**

(12) **EUROPEAN PATENT SPECIFICATION**

(45) Date of publication and mention
of the grant of the patent:
**28.06.2006 Bulletin 2006/26**

(51) Int Cl.:
*C12N 15/55* (2006.01)        *C12N 9/20* (2006.01)
*C07K 19/00* (2006.01)        *C12N 15/80* (2006.01)
*C12N 1/15* (2006.01)         *A21D 8/04* (2006.01)

(21) Application number: **04075819.5**

(22) Date of filing: **03.04.1998**

(54) **Lipase and use of same for improving doughs and baked products**

Verwendung von Lipase zur Verbesserung von Teigen Und Backwaren

Utilisation de lipase pour améliorer des pates à pain et des produits de boulangerie

(84) Designated Contracting States:
**AT BE CH CY DE DK ES FI FR GB GR IE IT LI LU MC NL PT SE**

(30) Priority: **09.04.1997 DK 40097**

(43) Date of publication of application:
**13.10.2004 Bulletin 2004/42**

(83) **Declaration under Rule 28(4) EPC (expert solution)**

(60) Divisional application:
**06002232.4**

(62) Document number(s) of the earlier application(s) in accordance with Art. 76 EPC:
**98913539.7 / 0 977 869**

(73) Proprietor: **DANISCO A/S**
**1001 Copenhagen K. (DK)**

(72) Inventors:
• **Poulsen, Charlotte H.**
**8220 Braband (DK)**
• **Soe, Jorn B.**
**8381 Mundelstrup (DK)**

• **Rasmussen, Preben**
**4070 Kirke Hyllinge (DK)**
• **Madrid, Susan M.**
**3500 Vaelose (DK)**
• **Zargahi, Masoud R.**
**8239 Abyhoj (DK)**

(74) Representative: **Williams, Aylsa**
**D Young & Co**
**120 Holborn**
**London EC1N 2DY (GB)**

(56) References cited:
**WO-A-94/04035            US-A- 5 232 846**

• **DIEGO COLOMBO ET AL.: "Optically pure 1-O- and 3-O-Beta-D-Glucosyl- and Galactosyl-sn-glycerols through lipase-catalyzed transformations" TETRAHEDRON LETTERS, vol. 36, no. 27, 1995, pages 4865-4868, XP004027788**
• **ATSUSHI TSUCHIYA ET AL.: "Cloning and nucleotide sequence of the mono- and diacylglycerol lipase gene (mdlB) of Aspergillus oryzae" FEMS MICROBIOLOGY LETTERS, vol. 143, 1996, pages 63-67, XP001053364**

**Description**

FIELD OF THE INVENTION

The present invention relates to the field of food manufacturing, in particular to the preparation of improved bakery products. Specifically, the invention provides a method for preparing a baked product having improved characteristics, comprising adding a polypeptide to a dough, wherein the polypeptide has lipase activity which is capable of conferring improved characteristics to food products including bakery products.

BACKGROUND OF THE INVENTION AND PRIOR ART

Lipases (EC 3.1.1.3), which can be defined as carboxylesterases which catalyze the hydrolysis of acylglycerols, are physiologically very important enzymes as one of the three major digestive enzymes together with amylases and proteases. They hydrolyse lipids to glycerol and fatty acids, but can also function in esterification or transesterification reactions.

Several studies report on the purification and characterisation of lipases from *Aspergillus niger.* Thus, Tombs and Blake (Biochim. Biophys., 1982, 700:81-89) purified a lipase from a commercial crude *Aspergillus* medium concentrate. The pure lipase was a glycosylated dimer containing two chains each having a molecular weight of 25 kDa.

Iwai and Tsujisaka (in Lipases, Borgström and Brockman (eds.), Elsevier, Amsterdam, 1984, pp 443-468) also purified an extracellularly secreted lipase from *Aspergillus niger* and obtained crystals of the lipase. They determined the molecular weight of the lipase to be 38 kDa and found that the enzyme was monomeric. The pI was determined to be 4.3. Optimum pH on olive oil was 5.6 and the optimum temperature on the same substrate was 25°C. The lipase was stable in a pH range of from 2.2 to 6.8 (30°C, 24 hours) and up to a temperature of 50°C (pH 5.6, 15 min). The lipase displayed a high activity towards triglycerides of medium chain length fatty acids.

Höfelmann et al. (J. Food Sci., 1985, **50**:1721-1731) used a commercial *Aspergillus niger* lipase product (Lipase 2212, Röhm) as starting material for purification of lipase. Two lipases with molecular weight 19 kDa and 31 kDa and a pI of 3.5 and 4.0, respectively were obtained. Both enzymes were glycosylated.

Torossian and Bell (Biotechnol. Appl. Biochem., 1991, **13**:205-211) used a crude commercial lipase preparation from *Aspergillus niger* from Amano (Japan). They determined the molecular weight to be 37 kDa and pI was 4.0. The N-terminal was determined to be XVSTSTLDELQFALQ. Sugihara et al. (Agric. Biol. Chem., 1988, **52**:1591-1592) found the N-terminal to be SVT, and the molecular weight to be 35 kDa for a lipase purified from an Amano (Japan) *Aspergillus niger* lipase preparation.

Despite the discrepancies in molecular weight for the mentioned lipases they were all reported to be 1,3-specific with regard to the hydrolysis.of triglycerides.

Within the baking industry it is well known to use enzymes, such as amylases, xylanases, oxidases and proteases, for the improvement of the dough; the dough handling properties and/or the baked product to obtain increased volume, retarded staling and greater softness. The use of lipases as baking additive is also known.

Thus, US 3,368;903 discloses purified lipase preparations isolated from plant seeds which, when added to a bread dough mixture, has a significant bread staling retarding effect.

JP-62-285749-A describes a method of bread making in which lipase is added to the dough in admixture with vital gluten and lecithin. It is stated that this lipase deteriorates quality properties such as bread volume and elasticity of the crumb.

Mohsen et al. (Egypt. J. Food Sci., 1986, **14**:175-182) describes that a lipase produced by *Rhizopus delemar* improves the softness of bread.

A bread improver comprising glucose oxidase in combination with oxidases other than glucose oxidase or hydrolases such as for example lipase is disclosed in EP 468 731 A1. There is obtained bread of a sufficient volume which is satisfactory in the quality of the internal and external characteristics. However, the use of lipase alone has a bread volume effect.

WO-94/04035 discloses a method of improving the properties of a dough (with and without added fat) and/or a baked product made from the dough by adding a lipase of microbial origin to the dough. The use of the microbial lipase resulted in an increased volume and improved softness of the baked product. Furthermore, an improved anti-staling effect was found.

EP 585 988 A1 discloses a bread improver composition comprising at least one lipase, at least one hemicellulase and at least one amylase. Baking experiments showed that the use of lipase alone in a dough without added fat resulted in a reduced volume of the baked product whereas no volume effect was observed when lipase is used in a dough containing added fat.

From the prior art it can thus be derived that the effects of known lipases when used as dough additives are highly variable in respect of antistaling or crumb firmness retardation and bread volume.

The present invention provides a method of preparing a baked product having improved pore homogeneity and reduced pore diameter comprising adding a polypeptide to a dough, wherein the polypeptide has lipase activity and is a triacylglycerol hydrolysing enzyme, which is capable of splitting off fatty acids having short, medium and long chain length, characterised in that said polypeptide is capable of hydrolysing galactolipids that are normally present in the flour to the corresponding galactosyl monoglycerides and wherein said polypeptide is capable of hydrolising at least 10% of the galactosyl diglycerides normally present in the flour dough to galactosyl monoglycerides and wherein said polypeptide retains at least 60% of its activity after 1 hour at 60°C in 100mM sodium acetate buffer at pH5.0. Novel polypeptides having lipase activity which were found to confer highly desirable characteristics not disclosed in the prior art to doughs and bakery products may be used in the method of the present invention. Thus, in baking experiments these polypeptides showed surprising and not previously taught or suggested properties when used in flour doughs, including increased crumb pore homogeneity and reduced pore diameter without concomitant negative effects on bread volume and crumb porosity. Thus, the method according to the invention provides baked products being less prone to mechanical deformation.

Small average pore diameter, increased pore homogeneity and unchanged porosity imply that the invention provides the means of obtaining baked products with a reinforced crumb structure. The improved pore homogeneity of the baked product further implies the advantage that there is obtained a product which is more sliceable and resistant to physical handling due to the reinforced crumb structure.

It is well known that it is difficult to spread thin layers of butter or margarine onto slices of bread having a very inhomogeneous pore structure. Therefore, it is an advantage that bread, as can be obtained in accordance with the present invention, has a fine and homogeneous pore structure.

It is well-known that a sliced loaf is less resistant to physical handling than un-sliced loaf. Therefore, the reinforced crumb structure, which is obtained by the method of the present invention, is particularly advantageous in baked products, such as toast bread, which are typically sliced immediately after baking by the manufacturer and are distributed in sliced condition to fast-food shops and supermarkets.

It has further been found that the polypeptide for use in the method of the present invention improves the stability of the gluten network in flour doughs which implies the advantage that the tolerance to variations in fermentation time is enhanced.

It is therefore an important objective of the present invention to provide a method comprising the use of such lipase active polypeptides. It has been found that such polypeptides may be derived from filamentous fungi such as e.g. *Aspergillus tubigensis.* However, the polypeptide is only produced in small amounts in wild-type fungal strains. Therefore a method of producing the polypeptides for use in the method of the present invention in a cost-effective manner by using recombinant DNA technology is also taught herein.

SUMMARY OF THE INVENTION

Accordingly, the present invention relates to a method of preparing a baked product having improved pore homogeneity and reduced pore diameter comprising adding a polypeptide to a dough, wherein the polypeptide has lipase activity and is a triacylglycerol hydrolysing enzyme, which is capable of splitting off fatty acids having short, medium and long chain length, characterised in that said polypeptide is capable of hydrolysing galactolipids that are normally present in the flour to the corresponding galactosyl monoglycerides and wherein said polypeptide is capable of hydrolysing at least 10% of the galactosyl diglycerides normally present in the flour dough to galactosyl monoglycerides and wherein said polypeptide retains at least 60% of its activity after 1 hour at 60°C in 100mM sodium acetate buffer at pH5.0. A polypeptide for use in the method of the present invention having lipase activity that is derivable from *Aspergillus tubigensis*, the polypeptide having the following characteristics: (i) it retains at least 80% activity after 4 days at 20°C at a pH in the range of 3.5-8, (ii) it retains at least 60% of its activity after 1 hour at 60°C in 100 mM sodium acetate buffer at pH 5.0, and (iii) it has an isoelectric point as determined by isoelectric focusing in the range of 3.5-4.5. Specifically, the polypeptide is one that comprises at least one amino acid sequence selected from the group consisting of SEQ ID NO:1, SEQ ID NO:2 and SEQ ID NO: 3, where Xaa in said sequences is an amino acid selected from the group consisting of Ala, Arg, Asn, Asp, Cys, Gln, Glu, Gly, His, Ile, Leu, Lys, Met, Phe, Pro, Ser, Thr, Trp, Tyr and Val.

A recombinant DNA molecule comprising a nucleotide sequence coding for the above lipase active polypeptide is also taught herein.

A Cell comprising the recombinant DNA molecule that is capable of expressing the polypeptide having lipase activity is also referred to herein by way of reference.

There is also taught herein a method of preparing the polypeptide for use in the method according to the invention, the method comprising transforming a host cell with a recombinant DNA molecule comprising a sequence coding for the polypeptide, the host cell is capable of expressing the nucleotide sequence coding for the polypeptide, cultivating the transformed host cell under conditions where the nucleotide sequence is expressed and harvesting the polypeptide.

Thus, the present invention relates in other aspects to a method for preparing a baked product having improved characteristics, comprising the use of the polypeptide having lipase activity in a dough for a baked product to impart improved pore homogeneity or reduced pore diameter to the baked product and a dough improving composition comprising the polypeptide and at least one further conventional dough additive component.

DETAILED DISCLOSURE OF THE INVENTION

As it is mentioned above, the invention relates in a first aspect to a polypeptide having lipase activity. As used herein the term "lipase" is used to designate any triacylglycerol hydrolysing enzyme, including such enzymes that are capable of splitting off fatty acids having short, medium and long chain length. According to the invention, the lipase active polypeptide is one which retains at least 80% activity after 4 days at 20°C and at a pH in the range of 3.5-8 including a pH in the range of 5-7.

To be practically useful, it is also advantageous that the polypeptide for use in the method of the invention has a good thermotolerance and optimum temperature for activity, at least to an extent where it is fully active in a dough at least up to the proofed dough is heated in an oven. Preferably, the lipase has a thermostability which renders the enzyme active during at least part of the baking process. Specifically, the thermostability of the polypeptide is at a level where it retains at least 60% of its activity after 1 hour at 60°C in 100 mM sodium acetate buffer at pH 5.0, including a polypeptide that retains at least 80% of its activity after 1 hour at 50°C under the same conditions.

In one specific embodiment, the polypeptide for use in the method of the invention comprises at least one amino acid sequence shown herein as SEQ ID NO:1, SEQ ID NO:2 and SEQ ID NO:3.

In advantageous embodiments, the polypeptide for use in the method according to the invention shows enzymatic activity at a pH in the range of 3.5-8.0, including the range of 5-7.

The polypeptide for use in the method according to the present invention has an isoelectric point determined by isoelectric focusing in the range of 3.5-4.5 such as the range of 3.8 to 4.2 including an isoelectric point of $4.0\pm0.1$.

A highly advantageous characteristic of the polypeptide for use in the method according to the invention is its capability to hydrolyse galactolipids such as galactosyl diglycerides, including digalactosyl diglyceride and monogalactosyl diglyceride, that are normally present in a flour, to the corresponding galactosyl monoglycerides. Thus, it has been found that the present polypeptides are capable of hydrolysing at least 10% of the galactosyl diglycerides-normally present in a flour dough to-monoglycerides. Preferably, at least 15% of these diglycerides, such as at least 25%, are hydrolysed.

The polypeptide for use in the method according to the invention can be in a glycosylated form. However, it has been found that the enzymatic activity of such a glycosylated lipase may be enhanced by deglycosylation. Accordingly, it may be preferred to provide the polypeptide in a non-glycosylated form. Thus, when the polypeptide fe obtained from its natural source or from a recombinant host cell in a glycosylated form, its activity can be enhanced by N-deglycosylation by at least partially removing carbohydrate moieties by digestion with a deglycosylating enzyme such as endo-β-N-acetyl-glucosamidase H.

Alternatively, a non-glycosylated polypeptide for use in the method of the invention is provided by means of modifying a DNA sequence coding for such polypeptide so as to provide a coding sequence that does not code for amino acids or a subsequence of the polypeptide that provides glycosylation sites. As it will be explained in the following, such mutated sequences coding for mutant polypeptides having lipase activity can be provided which, relative to the wild-type polypeptide, have an enhanced enzymatic activity.

The degree of glycosylation of the polypeptide as obtained is reflected in the molecular weight. As an example, when the polypeptide for use in the method of the invention is derived from *Aspergillus tubigensis* in a glycosylated form, it typically has a molecular weight as determined by gel filtration using Superose 12 of $3.2\pm1$ kDa. By matrix-assisted laser desorption ionisation mass spectrometry (MALDI-MS) the polypeptide for use in the method according to the invention typically has a molecular weight of $31\pm1.5$ kDa. It has been found that in this initially glycosylated polypeptide, N-linked oligosaccharides account for about 10% of the polypeptide.

In one specific embodiment , the polypeptide for use in the method according to the invention comprises the amino acid sequence shown herein as SEQ ID NO: 9 or a variant, homologue or fragment hereof.

In the present context, the terms "variant" or "fragment" in relation to the polypeptide for use in the method of the present invention include any substitution, variation, modification, replacement, deletion or addition of one or more amino acids from or to the SEQ ID NO:9 sequence provided the resultant amino acid sequence has lipase activity, preferably at least the same, activity as the polypeptide shown as SEQ ID NO:9.

In particular, the term "homologue" is used herein to include polypeptides having lipase activity which, relative to the sequence shown as SEQ ID NO:9, is of a similar amino acid composition or sequence, allowing for minor variations which do not have an adverse effect on the enzymatic properties and/or biological function, or which may give interesting and useful novel properties or biological functions. The homologous polypeptide may be derived from any organisms or it may also be derived through the use of recombinant DNA techniques whereby a naturally occurring polypeptide is modified in its sequence. Preferably, a homologous polypeptide is one where there is at least 75%, more preferably at least 85% and most preferably at least 95% homology to the sequence shown as SEQ ID No. 9.

In another embodiment of the invention, the polypeptide is part of a fusion protein that comprises further enzymatically active sequences. Although it is possible to construct such chimeric polypeptides by post-translational modifications, it is generally preferred to provide such fusion proteins by recombinant DNA means where a host cell is transformed with a DNA sequence coding for the fusion product and having this product expressed as a chimeric protein. Examples of such additional enzymatic activity include proteolytic, amylolytic and hemicellulolytic activities.

When a polypeptide is obtained from a cell expressing the gene coding for the polypeptide it is generally in the form of a more or less crude preparation containing other (contaminating) enzymatic activities. In accordance with the invention, it is also possible to provide the polypeptide in a substantially pure form. Such a purified polypeptide can be obtained by subjecting a crude enzyme preparation to any conventional method for purifying polypeptides and proteins.

According to the invention, the polypeptide is obtainable from *Aspergillus tubigensis* as it is described in the following examples. However, it can be derived from any organism that produces such a polypeptide, including fungi, yeast species, Gram-negative and Gram-positive bacteria, plant cells or animal cells, including human cells.

As it is mentioned above, interesting properties of the polypeptide for use in the method of the invention is its capability to reduce the crumb pore diameter and to increase the pore homogeneity of the crumb of bread. In one specific embodiment, the polypeptide is one which, when it is added to a bread dough in an amount of 5,000 lipase units (LUS) per kg flour, reduces the average pore diameter of the crumb of the bread made from the dough by at least 10%, relative to a bread which is made from a bread dough without addition of the lipase. In another embodiment, the polypeptide is a polypeptide which when it is added to a bread dough in an amount of 5,000 LUS per kg flour, increases the pore homogeneity of the crumb of the bread made from the dough by at least 5%, relative to a bread which is made from a bread dough without addition of the lipase.

There is described herein for reference a recombinant DNA molecule comprising a nucleotide sequence coding for the polypeptide having lipase activity as it is described above.

Such a nucleotide sequence can be isolated from a natural source or it can be constructed as it is described in details in the below examples where such a coding sequence isolated from *Aspergillus tubigensis* and referred to as *lipA* is described in details. The nucleotide sequence can also be synthesised based on amino acid, sequences of a naturally occurring polypeptide exhibiting lipase activity.

In useful embodiments, the recombinant DNA molecule comprises a nucleotide sequence coding for a polypeptide exhibiting lipase activity which comprises at least one of the amino acid sequences, shown herein as SEQ ID NO: 1, SEQ ID No:2 and SEQ ID N0:3 or a nucleotide sequence coding for a polypeptide exhibiting lipase activity which comprises the amino acid sequence shown as SEQ ID. No. 9.

In further specific embodiments, the recombinant DNA molecule comprises at least one of SEQ, ID NO:4, SEQ ID NO:5, SEQ ID NO: 6 and SEQ ID NO:7 or at least the coding sequence of the nucleotide sequence shown as SEQ ID NO:8 or a variant, homologue or fragment thereof, or a sequence complementary thereto.

In the present context, the terms "variant" or "fragment" in relation to the nucleotide sequence coding for the polypeptide of the present invention include any substitution of, variation of, modification of, replacement of, deletion of or addition of one or more nucleic acids from or to the sequence providing the resultant nucleotide sequence coding for a polypeptide having lipase activity, preferably having at least the same activity as the polypeptide shown as SEQ ID NO:9.

The term "homologue" covers homology with respect to sequence and/or structure and/or function providing the resultant nucleotide sequence codes for a polypeptide having lipase activity. With respect to sequence homology (i.e. similarity), preferably there is at least 75%, more preferably at least 85%, most preferably at least 95% homology to the sequence shown as SEQ ID NO:8.

The recombinant DNA molecule may advantageously comprise a sequence that codes for a polypeptide according to invention which does not comprise amino acid(s) providing glycosylation site(s). Such useful recombinant DNA molecules can be selected from the plasmids deposited under the accession Nos. NCIMB 40931, NCIMB 40932, NCIMB 40933, NCIMB 40934 and NCIMB 40935.

A cell comprising a recombinant DNA molecule as described above and which is capable of expressing the polypeptide for use in the method according to the invention is also taught herein. Such a cell can be selected from fungi, yeast species, bacteria, plant cells and animal cells including human cells. Useful cells are selected from filamentous fungi such as an *Aspergillus* sp., a Penicillium sp., a *Rhizomucor* sp., a *Mucor* sp., a *Trichoderma* sp. including *T. reesei*, *T. viridae* and *T. longibrachiatum*, a *Neurospora* sp. and a Humicola sp. Suitable *Aspergillus* species include *A. niger*, *A. tubigensis, A. oryzae* and *A. awamori.*

Useful bacterial host cells include Gram-negative species such as e.g. E. coli including the E. coli strain harbouring plasmid pLIP4 as deposited under the accession No. NCIMB 40863, and Gram-negative species such as e.g. *Bacillus* species and lactic acid bacterial species such as *Lactococcus lactis.*

A method of preparing. the polypeptide for use in the method according to the invention by expression of a nucleotide sequence capable of expressing the polypeptide in an appropriate transformed host cell is also taught herein.

The method comprises in a first step that a suitable transformable cell is transformed with the above recombinant DNA molecule to provide a transformed host cell expressing the polypeptide. Procedures for transformation of prokaryotic host cells is well documented in the art, for example see Sambrook et al. (Molecular Cloning: A Laboratory Manual, 2nd edition, 1989, Cold Spring Harbor Laboratory Press).

The term "transformed host cell" is used herein to include any transformable organism wherein the nucleotide sequence coding for the enzyme according to the present invention has been introduced. The introduction of the coding sequence can be in the form of introducing an episomal replicon such as a plasmid, a bacteriophage or a cosmid into the cell. Such replicons can advantageously be introduced in multiple copies to obtain an increased expression of the polypeptide. It may in certain cases be advantageous that the nucleotide sequence is incorporated into the genome of the host cell organism e.g. by means of a transposable element or a recombinational event.

A presently preferred liost organism for the expression of the nucleotide sequence of the present invention and/or for the preparation of the polypeptide for use in the method of the present invention is an organism of the genus *Aspergillus,* such as *Aspergillus niger* or *Aspergillus tubigensis.*

A transformed *Aspergillus* strain can e.g. be prepared according to methods described by Rambosek and Leach (CRC Crit. Rev. Biotechnol., 1987, 6:357-393); Davis (in: Progress in Industrial Microbiology, Martinelli and Kinghorn (eds.), Elsevier Amsterdam, 1994, 29:525-560), Ballance (in: Molecular Industrial Mycology, Systems and Applications for Filamentous Fungi, Leong and Berka (eds.), Marcel Dekker Inc., New York 1991, pp 1-29) and Turner (in: Progress in Industrial Microbiology, Martinelli and Kinghorn (eds.), Elsevier Amsterdam, 1994, 29:641-666).

In another embodiment, the method makes use of a yeast host cell such as *Saccharomyces cerevisiae* or *Pichia pastoris.* Expression of heterologous genes in *Saccharomyces cerevisiae* has been reviewed by Goody et al. (in: Yeast Biotechnology, Berry et al. (eds.), Allen and Unwin, London, 1987, pp 401-429) and by King et al. (in: Molecular and Cell Biology of Yeasts, Walton and Yarronton (eds.), Blackie, Glasgow, 1989, pp 107-133).

Furthermore, the present invention relates in a particular aspect to the use of the polypeptide according to the invention to improve the stability of the gluten network in a flour dough and to impart improved pore homogeneity and reduced pore diameter to the baked product made from said dough. It has been found that the polypeptide has these improving effects in a fat-free dough.

In the present context, the term "fat-free dough" is used to indicate that no lipid or fat is added to the flour dough. A preferred flour is wheat flour or a composite flour wherein part of the wheat flour is replaced by starch, optionally supplemented with plant protein and additives such as emulsifiers. Other types of flour derived from rice, maize, barley and rye are also contemplated.

Thus, the major ingredients of the dough include flour, preferably wheat flour, water and an "gas generating substance" such as yeast or a chemical leavening agent. In addition to the above-mentioned major ingredients the dough may include minor ingredients such as salt, sugar, minerals, vitamins, flavouring and at least one further dough additive such as for example an emulsifying agent, a hydrocolloid, a starch degrading enzyme or a cellulose or hemicellulose degrading enzyme.

During mixing and moulding of the dough ingredients to provide a homogeneous dough the interaction between wheat gluten, starch and water is essential for obtaining a dough with good dough handling properties and a satisfactory baked product made from the dough.

It is generally assumed that starch and gluten form a structural network including the glycolipids in the form of liquid-crystalline phases of lamellar structure as layers between gluten protein and starch.

The crumb structure of a baked product can, be evaluated by visual inspection of the bread cross section. However, a more reliable method giving a quantitative measure of the crumb structure is by image analysis using an image analyzer which on the entire cross, section of bread separates and analyses the individual pores one by one and calculate the mean pore size diameter. The bread cross section in its entirety is characterised by the distribution of the individual pores, for example in the form of a histogram. By homogeneity is understood the uniformity of the pore size and in the present context the term "homogeneity" is defined as the percentage of pores that are larger than 0..5 times the average of, pore diameter and smaller than 2 times the average pore diameter. The image analyzer also calculates the porosity which is the proportion of the entire bread cross section consisting of pores.

By using image analysis it has been found that baked products made from a dough as defined above including a fat-free dough, which has been supplemented by addition of the polypeptide for use in the method of the present invention attain/obtain an increased pore homogeneity and reduced pore size whereas the porosity is unchanged.

Thus, by using the polypeptide for use in the method according to the invention in a fat-free dough there is provided baked products with a fortified crumb structure. It has also been found that the increased pore homogeneity and the decrease in pore size is not accompanied by a reduction of other bread characteristics such as bread volume and anti-staling properties.

As already mentioned, a most interesting characteristic of the polypeptide for use in the method according to the present invention is its ability to modify by hydrolysis the glycolipids, monogalactosyl diglyceride (MGDG) and digalactosyl diglyceride (DGDG), to the more polar components monogalactosyl monoglyceride (MGMG) and digalactosyl monoglyceride (DGMG) which are more surface active components than MGDG and DGDG.

Without being bound by theory, it is assumed that the improved pore homogeneity of the bread crumb which is obtained by using the polypeptide of the invention in a dough is caused by the formation of the more surface active glycerides MGMG and DGMG which in combination with the released fatty acids in ionised form will contribute to the formation of mesomorphic phases of lamellar structure.

In accordance with the invention the amount of polypeptide added to the dough corresponds to a lipase activity in the range of 100-30,000 lipase units (LUS) per kg flour, such as in the range of 500-10,000 lipase units (LUS) per kg flour including in the range of 1,000-8,000 lipase units (LUS) per kg flour.

In an interesting embodiment, the method of the invention involves combined use of the polypeptide and an emulsifier such as mono- and diglycerides, sorbitan esters, polysorbates, sucrose esters, citric acid esters of mono- and diglycerides, polyglycerol esters of fatty acids, propylene glycol monostearate, lactic acid esters, lecithins, mono- and diglycerides of edible fatty acids and diacetyl tartaric acid ester of mono- and diglycerides of edible fatty acids

Diacetyl tartaric acid esters of mono- and diglycerides of edible fatty acids are well-known in the food processing technology and are widely used in the baking industry as dough additives to provide dough stability and increased volume of the baked products. Typically, diacetyl tartaric acid esters of mono- and diglycerides are used in an amount of up to 1% by weight of the flour.

It has been found that by using the polypeptide for use in the method of the invention in combination with diacetyl tartaric acid esters of mono- and diglycerides of fatty acids an improved pore homogeneity is still obtained and furthermore, that a much lower concentration of diacetyl tartaric acid esters of mono- and diglycerides of fatty acids is required to obtain the same bread volume.

According to the invention an expedient amount of diacetyl tartaric acid esters of mono- and diglycerides of fatty acids is in the range of 0.1 to 1.0% by weight of the flour, preferably in the range of 0.1 to 0.5% by weight of the flour and most preferred in the range of 0.1 to 0.4%.by weight of the flour.

The diacetyl tartaric acid esters of mono- and diglycerides which can be used according to the invention are characterised by having a saponification value in the range of 300 to 600, preferably a saponification value in the range of 300 to 400, and an acid value in the range of 40 to 120, preferably an acid value in the range of 50 to 100.

In accordance with the above description of the use of the polypeptide in a flour dough including a fat-free dough, the present invention relates in a further aspect to a method of preparing a baked product having improved pore homogeneity and reduced pore diameter from a dough including a fat-free dough as defined above, comprising adding the polypeptide of the invention to the dough.

In one embodiment the polypeptide having lipase activity has the capability of increasing tne level of ethylesters of fatty acids in a flour dough by at least 10% such as by least 50% including by at least 100%.

In accordance with the method of the invention, the polypeptide having lipase activity has the capability increasing the gluten index in a flour dough. Accordingly, in one useful embodiment, the method of preparing a baked product comprises adding to the dough the polypeptide in an amount that results in an increase of the gluten index, as determined by means of a Glutomatic 2200 apparatus, in the dough of at least 5%, relative to a dough without addition of the polypeptide.

Preferably, the gluten index is increased by at least 10% such as at least 15% or more preferably, by at least 20%.

In another useful embodiment, the present method is one wherein at least one further enzyme is added to the dough. Examples of such further enzymes include hemicellulases, proteases, amylases, oxidoreductases such as e.g. hexose oxidase and cellulases.

The polypeptide may conveniently be added to the dough or to any of the dough ingredients or to any mixture of the dough ingredients, in the form of a dry composition or as a liquid preparation comprising the polypeptide of the present invention.

As stated above the amount of polypeptide activity is in the range of 100-30,000 lipase units (LUS) per kg flour, including in the range of 500-10,000 lipase units (LUS) per kg flour such as in the range,of 1,000-8,000 lipase units (LUS) per kg flour.

In a useful embodiment of the method, the polypeptide of the invention is added to the dough in admixture with a diacetyl tartaric acid ester of mono- and diglycerides of edible fatty acids.

In another useful embodiment of the method the baked product is toast bread.

BRIEF DESCRIPTION OF THE FIGURES

The present invention is further illustrated by reference to the accompanying figures in which

Fig. 1 shows the restriction map of the genomic clone of the *lipA* gene,

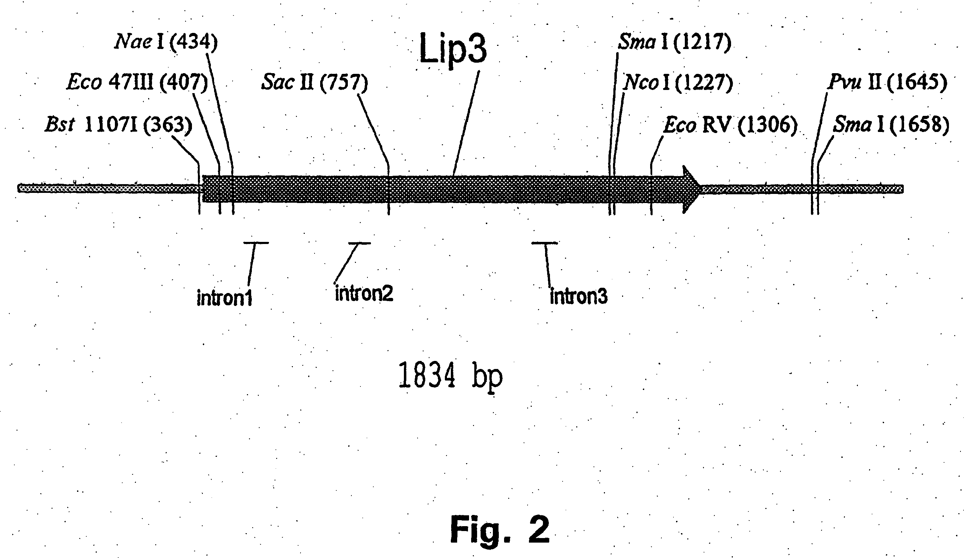

Fig. 2 shows the structure of the *lipA* gene encoding lipase 3,

Fig. 3 shows a chromatogram of HIC fractionated culture supernatant of an *Aspergillus tubigensis* transformant with 62 - fold increase of lipase 3, and

Fig. 4 shows a chromatogram of HIC fractionated culture supernatant of the untransformed *Aspergillus tubigensis* strain.

EXAMPLES

Analytical methods for determining lipase activity and protein

**(i) Determination of lipase activity**

1. Plate assay on tributyrin-containing medium

The assay is modified from Kouker and Jaeger (Appl. Environ. Microbiol., 1987, **53**:211-213).

A typical protocol for this assay is as follows: 100 ml 2% agar in 50 mM sodium phosphate buffer (pH 6.3) is heated to boiling, and after cooling to about 70°C under stirring, 5 ml 0.2% Rhodamine B is added under stirring plus 40 ml of tributyrin. The stirring is continued for 2 minutes. The mixture is then sonicated for 1 minute. After an additional 2 minutes of stirring, 20 ml of the agar mixture is poured into individual petri dishes. In the absence of lipase activity, the agar plates containing tributyrin and Rhodamine B will appear opaque and are pink coloured.

To quantify lipase activity, holes having a diameter of 3 mm are punched in the above agar and filled with 10 $\mu$l of lipase preparation. The plates are incubated for varying times at 37°C. When lipase activity is present in the applied preparation to be tested, a sharp pink/reddish zone is formed around the holes. When the plates are irradiated with UV light at 350 nm, the lipase activity is observed as halos of orange coloured fluorescence.

2. Modified-Food Chemical Codex assay for lipase activity

Lipase activity based on hydrolysis of tributyrin is measured according to Food Chemical Codex, Forth Edition, National Academy Press, 1996, p. 803. With the modification that the pH is 5.5 instead of 7. One LUT (lipase unit tributyrin) is defined as the amount of enzyme which can release 2 $\mu$mol butyric acid per min. under the above assay conditions.

3. *p*-nitrophenyl acetate assay

Lipase activity can also be determined colorimetrically using p-nitrophenyl acetate as a substrate e.g. using the following protocol : In a microtiter plate 10 $\mu$l of sample or blank is added followed by the addition of 250 $\mu$l substrate (0.5 mg p-nitrophenyl acetate per ml 50 mM phosphate buffer, pH 6.0). The microtiter plate; is incubated for 5 minutes at 30°C and the absorbance at 405 nm is read using a microplate reader. 1 unit is defined as 1 $\mu$mol *p*-nitrophenol released per 5 minutes.

4. *p*-nitrophenyl hexanoate assay

Lipase activity can be determined by using *p*-nitrophenyl hexanoate as a substrate. This assay is carried out by adding 10 $\mu$l of sample preparation or blank to a microtiter plate followed by the addition of 250 $\mu$l substrate (0.5 mg *p*-nitrophenyl hexanoate per ml of 20 mM phosphate buffer, pH 6.). At this concentration of substrate the reaction mixture appears as a milky solution. The microtiter plate is incubated for 5 minutes at 30°C and the absorbance at 405 nm is read in a microplate reader.

5. Titrimetric assay of lipase activity

Alternatively, lipase activity is determined according to Food Chemical Codex (3rd Ed., 1981, pp 492-493) modified to sunflower oil and pH 5.5 instead of olive oil and pH 6.5. The lipase activity is measured as LUS (lipase units sunflower) where 1 LUS is defined as the quantity of enzyme which can release 1 $\mu$mol of fatty acids per minute from sunflower oil under the above assay conditions.

6. Protein measurement.

During the course of purification of lipase as described in the following, the protein eluted from the columns was measured by determining absorbance at 280 nm. The protein in the pooled samples was determined in microtiter plates by a sensitive Bradford method according to Bio-Rad (Bio-Rad Bulletin 1177 EG, 1984). Bovine serum albumin was used as a standard.

EXAMPLE 1

Production, purification and characterization of lipase 3

1.1. Production

A mutant strain of *Aspergillus tubigensis* was selected and used for the production of wild type lipase. This lipase is referred to herein as lipase 3. The strain was subjected to a fermentation in a 750 1 fermenter containing 410.0 kg of tap water, 10.8 kg soy flour, 11.1 kg ammonium monohydrogenphosphate, 4.0 kg phosphoric acid (75%), 2.7 kg magnesium sulfate, 10.8 kg sunflower oil and 1.7 kg antifoam 1510. The substrate was heat treated at 121°C for 45 minutes. The culture media was inoculated directly with $7.5 \times 10^9$ spores of the mutant strain. The strain was cultivated for three days at 38°C, pH controlled at 6.5, aeration at 290 1/min and stirring at 180 rpm the first two days and at 360 rpm the last day. The fermentate was separated using a drum filter and the culture filtrate was concentrated 3.8 times by ultra-filtration. The concentrated filtrate was preserved with potassium sorbate (0.1%) and sodium benzoate (0.2%) and used as a starting material for purification of lipase.

1.2. Purification of lipase

A 60 ml sample of ferment (cf. 1.1) containing 557 LUS/ml, pH 5.5 was first filtered through a GF/B filter and subsequently through a 0.45 $\mu$m filter. The filtered sample was desalted using a Superdex G25 SP column (430 ml, 22 x 5 cm) equilibrated in 20 mM triethanolamine, pH 7.3. The flow rate was 5 ml/min. The total volume after desalting was 150 ml.
The desalted sample was applied to a Source Q30 anion exchanger column (100 ml, 5x5 cm) equilibrated in 20 mM triethanolamine, pH 7.3. The column was washed with equilibration buffer until a stable baseline was obtained. Lipase activity was eluted with a 420 ml linear gradient from 0 to 0.35 M sodium chloride in equilibration buffer, flow rate 5 ml/min. Fractions of 10 ml were collected. Sodium acetate (100 $\mu$l of a 2M solution) was added to each fraction to adjust pH to 5.5. Fractions 26-32 (70 ml) were pooled.
To the pool from the anion exchange step was added ammonium sulfate to 1 M and the sample was applied to a Source Phenyl HIC column (20 ml, 10x2 cm) equilibrated in 20 mM sodium acetate (pH 5.5), 1 M ammonium sulfate. The column was washed with the equilibration buffer. Lipase was eluted with a 320 ml linear gradient from 1 M to 0 M ammonium sulfate in 20 mM sodium acetate (pH 5.5), flow 1.5 ml/min. Fractions of 7.5 ml were collected.
Fractions 33-41 were analyzed by SDS-PAGE using a NOVEX system with precast gels. Both electrophoresis and silver staining of the gels were done according to the manufacturer (Novex, San Diego, USA). (The same system was used for native electrophoresis and isoelectric focusing). It was found that fraction 40 and 41 contained lipase as the only protein.

1.3. Characterization of the purified lipase

**(i) Determination of molecular weight**

The apparent molecular weight of the native lipase was 37.7 kDa as measured by the above SDS-PAGE procedure. The purified lipase eluted at a molecular weight of 32.2 kDa from a Superose 12 gel filtration column (50 mM sodium phosphate, 0.2 M sodium chloride, pH 6.85, flow 0.65 ml/min) and is therefore a monomer.
The molecular weight of the lipase was also determined by matrix-assisted laser desorption ionisation (MALDI) by means of a time-of-flight (TOF) mass spectrometer (Voyager Bio-Spectrometry Workstation, Perspective Biosystems). Samples were prepared by mixing 0.7 $\mu$l of desalted lipase solution and 0.7 $\mu$l of a matrix solution containing sinapic acid (3.5-dimethoxy-4-hydroxy cinnamic acid) in 70% acetonitrile (0.1% TFA, 10 mg/ml). 0.7 $\mu$l of the sample mixture was placed on top of a stainless steel probe tip and allowed to air-dry prior to introduction into the mass spectrometer. Spectra were obtained from at least 100 laser shots and averaged to obtain a good signal to noise ratio. The molecular mass for the lipase was found to be 30,384 Da and 30,310 Da by two independent analyses.
Digestion of the lipase with endo-$\beta$-N-acetyl-glucosamidase H (10 $\mu$l) from Streptomyces (Sigma) was carried out by adding 200 $\mu$l lipase and incubating at 37°C for 2 hours. The digestion mixture was desalted using a VSWP filter and analyzed directly by MALDI mass spectrometry. A major component of deglycosylated lipase gave a mass of 29,339 Da and 29,333 Da by two independent analyses. A minor component with a mass of 29,508 Da was also observed.

These values corresponds well to the later calculated theoretical value of 28,939 Da based.on the complete amino acid sequence of the mature lipase.

**(ii) Determination of the isoelectric point**

The isoelectric point (pI) for the lipase was determined by isoelectric focusing and was found to be 4.1.
A calculation of the pi based on the amino acid sequence as determined in the following and shown as SEQ ID NO: 9 gave an estimated pI of 4.07.

**(iii) Determination of temperature stability**

Eppendorf tubes with 25 $\mu$l of purified lipase 3 plus 50 $\mu$l 100 mM sodium acetate buffer (pH 5.0) were incubated for 1 hour in a water bath at respectively 30, 40, 50, and 60°C. A control was treated in the same way, but left at room temperature. After 1 hour the lipase 3 activity was determined by the p-nitrophenyl acetate assay as described above.
The purified lipase had a good thermostability. It was found that the lipase maintained 60% of its activity after 1 hour at 60°C. 80% and 85% activity was maintained after 1 hour at 50°C and 40°C respectively.

**(iv) Determination of pH stability**

Purified lipase 3 (200 $\mu$l) was added to 5 ml of 50 mM buffer solutions: (sodium phosphate, pH 8.0, 7.0 and 6.0 and sodium acetate pH 5.0, 4.0 and 3.5). The control was diluted in 5 ml of 4 mM sodium acetate pH 5.5. After four days at 20°C the residual activity was measured by the Modified Food Chemical Codex assay for lipase activity as described above. The lipase was very stable in the pH range from 4.0 to 7.0 where it maintained about 100% activity relative to the control (Table 1.1). At pH 3.5 the lipase maintained 92% activity, and at pH 8.0 95% residual activity was maitained as compared to the control.

Table 1.1. pH stability of lipase 3

| pH | Activity (LUT/ml) | Activity (%) |
|---|---|---|
| Control (pH 5.5) | 89.2 | 100 |
| 3.5 | 82.5 | 92 |
| 4.0 | 91.7 | 103 |
| 5.0 | 86.5 | 97 |
| 6.0 | 92.4 | 104 |
| 7.0 | 90.6 | 102 |
| 8.0 | 84.4 | 95 |

EXAMPLE 2

Amino acid sequencing of lipase 3

Purified lipase enzyme was freeze-dried and 100 $\mu$g of the freeze-dried material was dissolved in 50 $\mu$l of a mixture of 8 M urea and 0.4 M ammonium hydrogencarbonate, pH 8.4. The dissolved protein was denatured and reduced for 15 minutes at 50°C following overlay with nitrogen and addition of 5 $\mu$l 45 mM dithiothreitol. After cooling to room temperature, 5 $\mu$l of 100 mM iodoacetamide was added for the cysteine residues to be derivatized for 15 minutes at room temperature in the dark under nitrogen.
135 $\mu$l of water and 5 $\mu$g of endoproteinase Lys-C in 5 $\mu$l of water was added to the above reaction mixture and the digestion was carried out at 37°C under nitrogen for 24 hours. The resulting peptides were separated by reverse phase HPLC on a VYDAC C18 column (0.46 x 15 cm; 10 $\mu$m; The Separation Group, California, USA) using solvent A: 0.1% TFA in water and solvent B: 0.1% TFA in acetonitrile. Selected peptides were rechromatographed on a Develosil C18 column (0.46 x 10 cm, Novo Nordisk, Bagsvaerd, Denmark) using the same solvent system, prior to N-terminal sequencing. Sequencing was done using an Applied Biosystems 476A sequencer using pulsed-liquid fast cycles according to the manufacturer's instructions (Applied Biosystems, California, USA).
For direct N-terminal sequencing, the purified protein was passed through a Brownlee C2 Aquapore column (0.46 x 3 cm, 7 $\mu$m, Applied Biosystems, California, USA) using the same solvent system as above. N-terminal sequencing was then performed as described above. As the protein was not derivatized prior to sequencing, cysteine residues could not be determined.

The following peptide sequences were found:

| N-terminal: | Ser-Val-Ser-Thr-Ser-Thr-Leu-Asp-Glu-Leu-Gln-Leu-Phe-Ala-Gln-Trp-ser-Ala-Ala-Ala-Tyr-X-Ser-Asn-Asn (SEQ ID NO:1) |
| Internal peptide 1: | Val-His-Thr-Gly-Phe-Trp-Lys (SEQ ID NO:2). |
| Internal peptide 2: | Ala-Trp-Glu-Ser-Ala-Ala-Asp-Glu-Leu-Thr-Ser-Lys-Ile-Lys, (SEQ ID NO:3) |

No further peptides could be purified from the HPLC fractionation assumingly because they were very hydrophobic and therefore tightly bound to the reverse phase column.

A search in SWISS-PROT database release 31 for amino acid sequences with homology to the above peptides was performed and only three sequences were found.

All of the above peptides showed a low homology to the above known sequences. Especially internal peptide 2 has very low homology to the three lipases, LIP-RHIDL, LIP-RHIMI and MDLA-PENCA from *Rhizopus delamar* (Haas and Berka, Gene, 1991, 109:107-113), *Rhizomucor miehei* (Boel et al., Lipids, 1988, 23:701-706) and Penicillium camenbertii (Yamaguchi et al., Gene, 1991, 103:61-67; Isobe and Nokihara, Fobs. Lett., 1993, 320:101-106) respectively. Although the homology was not very high it was possible to position the lipase 3 peptides on these sequences as it is shown in the below Table 2.1.

## Table 2.1. Alignment of lipase 3 peptides with known lipase sequences

```
LIP_RHIDL    MVSFISISQGVSLCLLVSSMMLGSSAVPVSGKSGSSNTAVSASDNAALPP    50
LIP_RHIMI    MVLKQRANYLGFLIVFFTAFLV--EAVPIKRQSNSTVDS--------LPP    40
MDLA_PENCA   MRLS----------FFTAL----------------SAVASLGYALPG      21
             *                .  ...                .      **

N-Terminal               SVSTSTLDELQLFAQWSAAAYXSNN

LIP_RHIDL    LISSRCAPPSNKGSKSDLQAEPYNMQKNTEWYESHGGNLTSIGKRDDNLV   100
LIP_RHIMI    LIPSRTSAPSSSPSTTDPEAPAM---------SRNGPLPS----DVETK    76
MDLA_PENCA   KLQSR------DVSTSELDQFEFWVQYAAASY------------------    47
               .  **        .  *.....  ..

LIP_RHIDL    GGMTLDLPSDAPPISLSSSTNSASDGGKVVAATTAQIQEFTKYAGIAATA   150
LIP_RHIMI    YGMALNATSYPDSV-----VQAMSIDGGIRAATSQEINELTYYTTLSANS   121
MDLA_PENCA   -----------------------------------YEADYTAQVGDKL     60
                                                *  .  .  ....

LIP_RHIDL    YCRSVVPGNKWDCVQCQKWVPDGKIITTFT-SLLSDTNGYVLRSDKQKTI   199
LIP_RHIMI    YCRTVIPGATWDCIHCDA-TEDLKIIKTWS-TLIYDTNAMVARGDSEKTI   169
MDLA_PENCA   SCSKG------NCPEVEA--TGATVSYDFSDSTITDTAGYIAVDHTNSAV   102
             *..         .*  .      .  .. ...  . : **..  ........

Peptide 1                             VHTGFWK
Peptide 2                               AWESAADELTSK

LIP_RHIDL    YLVFRGTNSFRSAITDIVFNFSDYKPVKGAKVHAGFLSSYEQVVNDYFPV   249
LIP_RHIMI    YIVFRGSSSIRNWIADLTFVPVSYPPVSGTKVHKGFLDSYGEVQNELVAT   219
MDLA_PENCA   VLAFRGSYSVRNWVADATFVHTNPGLCDGCLAELGFWSSWKLVRDDIIKE   152
             ..***. * *. ..* .*    .  .* ..** ...  . ..
```

```
Peptide 2   IK


LIP_RHIDL    VQEQLTAHPTYKVIVTGHSLGGAQALLAGMDLYQREPRLSPKNLSIFTVG   299
LIP_RHIMI    VLDQFKQYPSYKVAVTGHSLGGATALLCALDLYQREEGLSSSNLFLYTQG   269
MDLA_PENCA   LKEVVAQNPNYELVVVGHSLGAAVATLAATDL--RGKGYPSAKLYAYA--   198
             . .   . *.. *.*****.* * * . ** *.   .. .*  ..


LIP_RHIDL    GPRVGNPTFAYYVESTGIPFQRTVHKRDIVPHVPPQSFGFLHPGVESWIK   349
LIP_RHIMI    QPRVGDPAFANYVVSTGIPYRRTVNERDIVPHLPPAAFGFLHAGEEYWIT   319
MDLA_PENCA   SPRVGNAALAKYITAQGNNF-RFTHTNDPVPKLPLLSMGYVHVSPEYWIT   247
             ****....* *.. * . * ...* **..*  ..*..* . * **.


LIP_RHIDL    SGTSN-V-----QICTSEIETKDCSNSIVPFTSILD-HLSYF-DINEGSC   391
LIP_RHIMI    DNSPETV-----QVCTSDLETSDCSNSIVPFTSVLD-HLSYF-GINTGLC   362
MDLA_PENCA   SPNNATVSTSDIKVIDGDVSFDGNTGTGLPLLTDFEAHIWYFVQVDAGKG   297
             . . . *       .. ....:. . ...:.*. . .. *. ** ...*


LIP_RHIDL    -------L   392
LIP_RHIMI    -------T   363
MDLA_PENCA   PGLPFKRV   305
```

EXAMPLE 3

Isolation and purification of *Aspergillus tubigensis* genomic DNA

The *Aspergillus tubigensis* mutant strain was grown in PDB (Difco) for 72 hours and the mycelium was harvested. 0.5-1 g of mycelium was frozen in liquid nitrogen and ground in a mortar. Following evaporation of the nitrogen, the ground mycelium was mixed with 15 ml of an extraction buffer (100 mM Tris-HCl, pH 8.0, 50 mM EDTA, 500 mM NaCl, 10 mM β-mercaptoethanol) and 1 ml 20 % sodium dodecylsulfate. The mixture was vigorously mixed and incubated at 65°C for 10 min. 5 ml 3M potassium acetate, (pH 5.1 adjusted with glacial acetic acid) was added and the mixture further incubated on ice for 20 min. The cellular debris was removed by centrifugation for 20 min. at 20,000 x g and 10 ml isopropanol was added to the supernatant to precipitate (30 min at -20°C) the extracted DNA. After further centrifugation for 15 min at 20,000 × g, the DNA pellet was dissolved in 1 ml TE (10 mM Tris·HCl pH 8.0, 1 mM EDTA) and precipitated again by addition of 0.1 ml 3 M NaAc, pH 4.8 and 2.5 ml ethanol. After centrifugation for 15 min at 20,000 x g the DNA pellet was washed with 1 ml 70 % ethanol and dried under vacuum. Finally, the DNA was dissolved in 200 μl TE and stored at -20°C.

EXAMPLE 4

The generation of a fragment of the putative gene coding for lipase 3 using PCR

To obtain a fragment of the putative gene (in the following referred to as the *lipA* gene) as a tag to isolate the complete gene, a PCR amplification procedure based on the information in the isolated peptide sequences was carried out.

Degenerated primers for PCR amplification of a fragment of the lipase gene were designed based on the amino acid sequences of the isolated peptides. The following three PCR primers were synthesised:

C035:        TTC CAR AAN CCN GTR TGN AC          (SEQ ID NO:4

20 mer 256 mixture, based on peptide 1 sequence VHTGFWK (Reversed).

C036:        CAR YTN TTY GCN CAR TGG        (SEQ ID NO:5)

18 mer 256 mixture, based on the N-terminal sequence QLFAQW.

C037:        GCV GCH SWY TCC CAV GC        (SEQ ID NO:6)

17 mer 216 mixture, based on internal peptide 2 sequence AWESAA (reversed).

The oligonucleotides were synthesised on a Applied Biosystems model 392 DNA/RNA Synthesizer. To reduce the degree of degeneracy the rare Ala codon GCA and the Ser codon TCA have been excluded in design of primer C037.

With these primers the desired fragments were amplified by PCR. Using these primers it was expected that a fragment of about 300 bp should be amplified provided there are no introns in the fragment.

The following PCR reactions were set up in 0.5 ml PCR tubes to amplify a putative *lip*A fragment:

1. 0.5 μg total genomic DNA,
100 pmol primer C036,
100 pmol primer C037,
10 μl PCR Buffer II (Perkin Elmer),
6 μl 25 mM $MgCl_2$,
2 μl dNTP mix (10 mM dATP, 10 mM dCTP, 10 mM dGTP, 10 mM dTTP),
2 units Amplitaq polymerase (Perkin Elmer), and
water to a total volume of 100 μl.

2. 0.5 μg total genomic DNA,
100 pmol primer C035,
100 pmol primer C036;
10 μl PCR Buffer II (Perkin Elmer),
6 μl 25 mM $MgCl_2$,
2 μl dNTP mix (10 mM dATP, 10 mM dCTP, 10 mM dGTP, 10 mM dTTP),
2 units Amplitaq polymerase (Perkin Elmer), and
water to a total volume of 100 μl.

The reactions were performed using the following program:

| | |
|---|---|
| 94°C | 2 min |
| 94°C | 1 min) |
| 40°C | 1 min) |
| 72°C | 1 min) These three steps were repeated for 30 |
| 72°C | 5 min cycles |
| 5°C | SOAK |

The PCR amplifications were performed in a MJ Research Inc. PTC-100 Thermocycler.

In reaction 1, three distinct bands of about 300, 360 and 400 bp, respectively could be detected. These bands were isolated and cloned, using the pT7-Blue-T-vector kit (Novagene). The sizes of these fragment is in agreement with the expected size provided that the fragment contains 0, 1 or 2 introns, respectively.

The three fragments were sequenced using a "Thermo Sekvenase fluorescent labelled primer cycle sequencing Kit" (Amersham) and analyzed on a ALF sequencer (Pharmacia) according to the instructions of the manufacturer. The fragment of about 360 bp contained a sequence that was identified as a lipase and, as it contained the part of the N-terminal distal to the sequence used for primer design, it was concluded that the desired *lip*A gene fragment was obtained.

The sequence of the about 360 bp PCR fragment (SEQ ID NO:7) is shown in the following Table 4:1. The peptide sequence used for primer design is underlined. The remaining part of the N-terminal sequence is doubly underlined.

## Table 4.1. PCR-generated putative *lip*A sequence

```
              10        20        30        40        50        60
               |         |         |         |         |         |
          tacccgggggntccgattCAGTTGTTCGCGCAATGGTCTGCCGCAGCTTATTGCTCGAATA

                          Q  L  F  A  Q  W  S  A  A  A  Y  C  S  N

              70        80        90       100       110       120
               |         |         |         |         |         |
          ATATCGACTCGAAAGAVTCCAACTTGACATGCACGGCCAACGCCTGTCCATCAGTCGAGG

           N   I  D  S  K  X  S  N  L  T  C  T  A  N  A  C  P  S  V  E

             130       140       150       160       170       180
               |         |         |         |         |         |
          AGGCCAGTACCACGATGCTGCTGGAGTTCGACCTGTATGTCACTCAGATCGCAGACATAG

           E  A  S  T  T  M  L  L  E  F  D  L  Y  V  T  Q  I  A  D  I

             190       200       210       220       230       240
               |         |         |         |         |         |
          AGCACAGCTAATTGAACAGGACGAACGACTTTTGGAGGCACAGCCGGTTTCCTGGCCGCG

           E  H  S  -  L  N  R  T  N  D  F  W  R  H  S  R  F  P  G  R

             250       260       270       280       290       300
               |         |         |         |         |         |
          GACAACACCAACAAGCGGCTCGTGGTCGCCTTCCGGGGAAGCAGCACGATTGAGAACTGG

           G  Q  H  Q  Q  A  A  R  G  R  L  P  G  K  Q  H  D  -  E  L

             310       320       330
               |         |         |
          ATTGCTAATCYTGACTTCATCCTGGRAGATAACG

           D  C  -  X  -  L  H  P  X  R  -
```

The finding of this sequence permitted full identification of the PCR fragment as part of the *lip*A gene. The stop codon found in the reading frame can be caused either by a PCR or a reading error or there can be an intron encoded in the fragment as a consensus intron start and ending signal (shown in bold). If the putative intron is removed a shift in reading frame will occur. However, an alignment of the deduced amino acid sequence and the fungal lipases shown in Table 2.1 the fragment was part of the desired gene.

EXAMPLE 5

Cloning and characterisation of the *lip*A gene

(i) Construction of an *Aspergillus tubigensis* genomic library

*Aspergillus tubigensis* genomic DNA was digested partially with Tsp5091 (New England Biolabs Inc.). 10 $\mu$g DNA was digested in 100 $\mu$l reaction mixture containing 2 units Tsp5091. After 5, 10, 15 and 20 minutes 25 $\mu$l was removed from the reaction mixture and the digestion was stopped by addition of 1 $\mu$l 0.5 M EDTA,pH 8.0. After all four reactions had been stopped, the samples were run on a 1% agarose gel in TAE buffer (10 x TAE stock containing per litre: 48.4 g Trizma base, 11.5 ml glacial acetic acid, 20 ml 0.5 M EDTA pH 8.0). HindIII-digested phage Lambda DNA was used as molecular weight marker (DNA molecular weight marker II, Boehringer, Mannheim). Fragments of a size between about 5 and 10 kb were cut out of the gel and the DNA fragments were purified using Gene Clean II Kit (Bio-101 Inc.). The purified fragments were pooled and 100 ng of the pooled fragments were ligated into 1 $\mu$g EcoRI-digested and dephosphorylated ZAP II vector (Stratagene) in a total volume of 5 $\mu$l. 2 $\mu$l of this volume was packed with Gigapack II packing extract (Stratagene) which gave a primary library of 650,000 pfu.

*E. coli* strain XL1-Blue-MRF (Stratagene) was infected with 5x 50,000 pfu of the primary library. The infected bacteria were mixed with top agarose (as NZY plates but with 6 g agarose per litre instead of the agar) and plated on 5 NZY plates (13 cm). After incubation at 37°C for 7 hours, 10 ml SM buffer (per litre: 5.8 g NaCl, 2.0 g MgCl$_2$·7H$_2$O, 50 ml 1 M Tris HCl pH 7.5, 5.0 ml of 2% (w/v) gelatine) and incubated overnight at room temperature with gently shaking. The buffer containing washed-out phages was collected and pooled. 5% chloroform was added and after vigorous mixing the mixture was incubated 1 hour at room temperature. After centrifugation for 2 minutes at 10,000 x g the upper phase containing the amplified library was collected and dimethylsulphoxide was added to 7%. Aliquots of the library was taken out in small tubes and frozen at -80°C. The frozen library contained 2.7 x 10$^9$ pfu/ml with about 6% without inserts.

(ii) Screening of the *Aspergillus tubigensis* library

2 x 50.000 pfu were plated on large (22 x 22 cm) NZY plates containing a medium containing per litre: 5 g NaCl, 2 g MgSO$_4$·7H$_2$O, 5 g yeast extract, 10 g casein hydrolysate, 15 g agar, pH adjusted to 7.5 with NaOH. The medium was autoclaved and cooled to about 60°C and poured into the plates. Per plate was used 240 ml of medium.

The inoculated NZY plates were incubated overnight at 37°C and plaque lifts of the plates were made. Two lifts were made for each plate on Hybond N (Amersham) filters. The DNA was fixed using UV radiation for 3 min. and the filters were hybridized as described in the following using, as the probe, the above PCR fragment of about 360 bp that was labelled with $^{32}$P-dCTP-using Ready-t-o-Go labelling kit (Pharmacia).

The filters were prehybridised for one hour at 65°C in 25 ml prehybridisation buffer containing 6.25 ml 20 x SSC (0.3 M Na$_3$citrate, 3 M NaCl), 1,25 ml 100 x Denhard solution, 1.25 ml 10% SDS and 16.25 ml water. 150 $\mu$l 10 mg/ml denatured Salmon sperm DNA was added to the prehybridization buffer immediately before use. Following prehybridization, the prehybridisation buffer was discarded and the filters hybridised overnight at 65°C in 25 ml prehybridisation buffer with the radiolabelled PCR fragment.

Next day the filters were washed according to the following procedure: 2 $\times$ 15 min. with 2 x SSC + 0.1.% SDS, 15 min. with 1 x SSC + 0.1 % SDS and 10 min. with 0.1 x SSC + 0.1% SDS.

All washes were done at 65°C. The sheets were autoradiographed for 16 hours and positive clones were isolated. A clone was reckoned as positive only if there was a hybridisation signal on both plaque lifts of the plate in question.

Seven putative clones were isolated and four were purified by plating on small petri dishes and performing plaque lifts essentially as described above.

The purified clones were converted to plasmids using an ExAssist Kit (Stratagene).

Two sequencing primers were designed based on the about 360 bp PCR fragment. The sequencing primers were used to sequence the clones and a positive clone with the *lipA* gene encoding lipase 3 was found. The isolated positive clone was designated pLIP4.

**(iii) Characterisation of the pLIP4 clone**

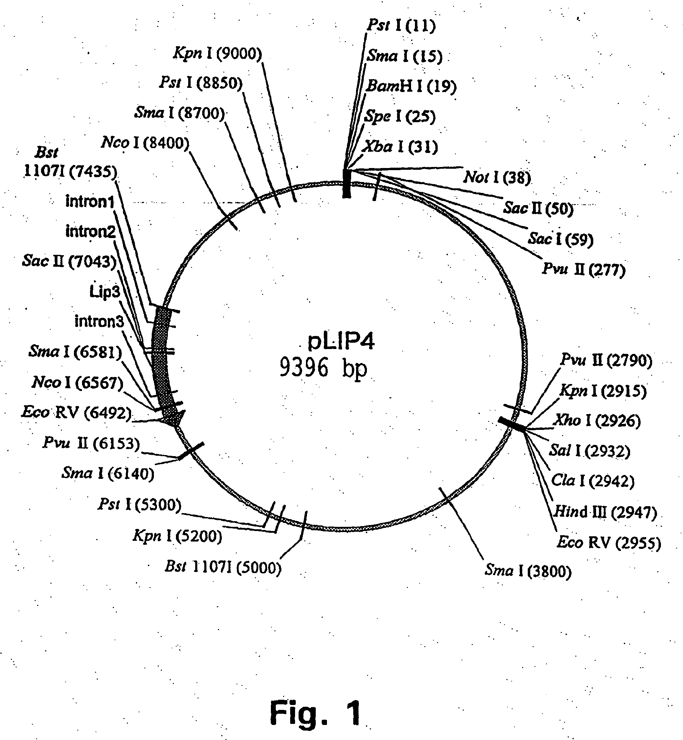

A restriction map of the clone was made. The above 360 bp PCR fragment contained a SacII site and as this site could be found in the genomic clone as well this site facilitated the construction of the map. The restriction map showing the structure of pLIP4 is shown in Fig. 1. The restriction map shows that the complete gene is present in the clone. Additionally, since promoter and terminator sequences are present, it was assumed that all the important regions is present in the clone.

A sample of *Escherichia coli* strain DH5α containing pLIP4 was deposited in accordance with the Budapest Treaty with The National Collections of Industrial and Marine Bacteria Limited (NCIMB) at 23 St. Machar Drive, Aberdeen, Scotland, United Kingdom, AB2 1RY on 24 February 1997 under the accession number NCIMB 40863.

The gene was sequenced using cycle sequencing and conventional sequencing technology. The complete sequence (SEQ ID NO:8) is shown below in Table 5.1. The sequence has been determined for both strands for the complete coding region and about 100 bp upstream and downstream of the coding region. The sequences downstream to the coding region have only been determined on one strand and contains a few uncertainties. In Table 5.1 shown below, the intron sequences are indicated as lowercase letters and the N-terminal and the two internal peptides (peptide 1 and peptide 2) are underlined:

Table 5.1. The DNA sequence for the *lipA* gene and flanking sequences

```
1     CCNDTTAATCCCCCACCGGGGTTCCCGCTCCCGGATGGAGATGGGGCCAAAACTGGCAAC
61    CCCCAGTTGCGCAACGGAACAACCGCCGACCCGGAACAAAGGATGCGGATGAGGAGATAC
121   GGTGCCTGATTGCATGGCTGGCTTCATCTGCTATCGTGACAGTGCTCTTTGGGTGAATAT
181   TGTTGTCTGACTTACCCCGCTTCTTGCTTTTTCCCCCCTGAGGCCCTGATGGGGAATCGC
241   GGTGGGTAATATGATATGGGTATAAAAGGGAGATCGGAGGTGCAGTTGGATTGAGGCAGT
301   GTGTGTGTGTGCATTGCAGAAGCCCGTTGGTCGCAAGGTTTTGGTCGCCTCGATTGTTTG
361   TATACCGCAAGATGTTCTCTGGACGGTTTGGAGTGCTTTTGACAGCGCTTGCTGCGCTGG
                    M   F   S   G   R   F   G   V   L   L   T   A   L   A   A   L
421   GTGCTGCCGCGCCGGCACCGCTTGCTGTGCGGAgtaggtgtgcccgatgtgagatggttg
      G   A   A   A   P   A   P   L   A   V   R
481   gatagcactgatgaagggtgaatagGTGTCTCGACTTCCACGTTGGATGAGTTGCAATTG
                                    S   V   S   T   S   T   L   D   E   L   Q   L
541   TTCGCGCAATGGTCTGCCGCAGCTTATTGCTCGAATAATATCGACTCGAAAGACTCCAAC
      F   A   Q   W   S   A   A   A   Y   C   S   N   N   I   D   S   K   D   S   N
601   TTGACATGCACGGCCAACGCCTGTCCATCAGTCGAGGAGGCCAGTACCACGATGCTGCTG
      L   T   C   T   A   N   A   C   P   S   V   E   E   A   S   T   T   M   L   L
661   GAGTTCGACCTgtatgtcactcagatcgcagacatagagcacagctaatttgaacagGAC
      E   F   D   L                                                             T
721   GAACGACTTTGGAGGCACAGCCGGTTTCCTGGCCGCGGACAACACCAACAAGCGGCTCGT
      N   D   F   G   G   T   A   G   F   L   A   A   D   N   T   N   K   R   L   V
781   GGTCGCCTTCCGGGGAAGCAGCACGATTGAGAACTGGATTGCTAATCTTGACTTCATCCT
      V   A   F   R   G   S   S   T   I   E   N   W   I   A   N   L   D   F   I   L
841   GGAAGATAACGACGACCTCTGCACCGGCTGCAAGGTCCATACTGGTTTCTGGAAGGCATG
      E   D   N   D   D   L   C   T   G   C   K   V   H   T   G   F   W   K   A   W
901   GGAGTCCGCTGCCGACGAACTGACGAGCAAGATCAAGTCTGCGATGAGCACGTATTCGGG
      E   S   A   A   D   E   L   T   S   K   I   K   S   A   M   S   T   Y   S   G
961   CTATACCCTATACTTCACCGGGCACAGTTTGGGCGGCGCATTGGCTACGCTGGGAGCGAC
      Y   T   L   Y   F   T   G   H   S   L   G   G   A   L   A   T   L   G   A   T
1021  AGTTCTGCGAAATGACGGATATAGCGTTGAGCTGgtgagtccttcacaaaggtgatggag
      V   L   R   N   D   G   Y   S   V   E   L
1081  cgacaatcgggaacagacagtcaatagTACACCTATGGATGTCCTCGAATCGGAAACTAT
                                    Y   T   Y   G   C   P   R   I   G   N   Y
1141  GCGCTGGCTGAGCATATCACCAGTCAGGGATCTGGGGCCAACTTCCGTGTTACACACTTG
      A   L   A   E   H   I   T   S   Q   G   S   G   A   N   F   R   V   T   H   L
```

```
1201 AACGACATCGTCCCCCGGGTGCCACCCATGGACTTTGGATTCAGTCAGCCAAGTCCGGAA
       N  D  I  V  P  R  V  P  P  M  D  F  G  F  S  Q  P  S  P  E
1261 TACTGGATCACCAGTGGCAATGGAGCCAGTGTCACGGCGTCGGATATCGAAGTCATCGAG
       Y  W  I  T  S  G  N  G  A  S  V  T  A  S  D  I  E  V  I  E
1321 GGAATCAATTCAACGGCGGGAAATGCAGGCGAAGCAACGGTGAGCGTTGTGGCTCACTTG
       G  I  N  S  T  A  G  N  A  G  E  A  T  V  S  V  V  A  H  L
1381 TGGTACTTTTTTGCGATTTCCGAGTGCCTGCTATAACTAGACCGACTGTCAGATTAGTGG
       W  Y  F  F  A  I  S  E  C  L  L  -
1441 ACGGGAGAAGTGTACATAAGTAATTAGTATATAATCAGAGCAACCCAGTGGTGGTGATGG
1501 TGGTGAAAGAAGAAACACATTGAGTTCCCATTACGKAGCAGWTAAAGCACKTKKGGAGGC
1561 GCTGGTTCCTCCACTTGGCAGTTGGCGGCCATCAATCATCTTTCCTCTCCTTACTTTCGT
1621 CCACCACAACTCCCATCCTGCCAGCTGTCGCATCCCCGGGTTGCAACAACTATCGCCTCC
1681 GGGGCCTCCGTGGTTCTCCTATATTATTCCATCCGACGGCCGACGTTTCACCCTCAACCT
1741 GCGCCGCCGCAAAATCTCCCCGAGTCGGTCAACTCCCTCGAACCGCCGCCCGCATCGACC
1801 TCACGACCCCGACCGTCTGYGATYGTCCAACCG
```

**(iv) Analysis of the sequence of the complete gene**

The peptide sequences obtained could all be found in the deduced amino acid sequence (see Table 5.1) which confirms again that the sequence found is the sequence of the lipase 3 gene. The gene was deeignated *lip*A.

The amino acid sequence was aligned with the three fungal lipases used to align the peptide sequences. The alignment is shown in Table 5.2.

## Table 5.2 Alignment of the lipase 3 sequence with known fungal lipases

```
LIPASE3      MFSG-----------RFGVLL-------------------------TALAA   15
MDLA_PENCA   MRLS-----------FFTAL--------------------------SAVAS   14
LIP_RHIDL    MVSFISISQGVSLCLLVSSMMLGSSAVPVSGKSGSSNTAVSASDNAALPP   50
LIP_RHIMI    MVLKQRANYLGFLIVFFTAFLV--EAVPIKRQSNSTVDS-------LPP   40
             *                  . .                          ...


LIPASE3      L--------------------------------------------------   16
MDLA_PENCA   L--------------------------------------------------   15
LIP_RHIDL    LISSRCAPPSNKGSKSDLQAEPYNMQKNTEWYESHGGNLTSIGKRDDNLV   100
LIP_RHIMI    LIPSRTSAPSSSPSTTDPEAPAM---------SRNGPLPS----DVETK   76
             *
```

```
LIPASE3     --------GAAAPAPLA-------------VRSVSTSTLDELQLFAQWSAAA      47
MDLA_PENCA  --------GYALPGKLQ-------------SRDVSTSELDQFEFWVQYAAAS      46
LIP_RHIDL   GGMTLDLPSDAPPISLSSSTNSASDGGKVVAATTAQIQEFTKYAGIAATA      150
LIP_RHIMI   YGMALNATSYPDSV------VQAMSIDGGIRAATSQEINELTYYTTLSANS      121
                          . . ..          .... ....  ..  .*...

LIPASE3     YCSNNIDSK-DSNLTCTANACPSVEEASTTMLLEFDLTNDFGGTAGFLAA       96
MDLA_PENCA  YYEADYTAQVGDKLSCSKGNCPEVEATGATVSYDFS-DSTITDTAGYIAV       95
LIP_RHIDL   YCRSVVP---GNKWDCVQ--CQKWVPDGKIIT---TFTSLLSDTNGYVLR      192
LIP_RHIMI   YCRTVIP---GATWDCIH--CDA-TEDLKIIK---TWSTLIYDTNAMVAR      162
            *.  .      ... .*   *     . ..   . ...*... .

LIPASE3     DNTNKRLVVAFRGSSTIENWIANLDFILEDNDDLCTGCKVHTGFWKAWES      146
MDLA_PENCA  DHTNSAVVLAFRGSYSVRNWVADATFV-HTNPGLCDGCLAELGFWSSWKL      144
LIP_RHIDL   SDKQKTIYLVFRGTNSFRSAITDIVFNFSDYKPV-KGAKVHAGFLSSYEQ      244
LIP_RHIMI   GDSEKTIYIVFRGSSSIRNWIADLTFVPVSYPPV-SGTKVHKGFLDSYGE      211
            ...... . ..***.  . .  ...   *  .    . .*  .. **  ...

LIPASE3     AADELTSKIKSAMSTYSGYTLYFTGHSLGGALATLGATVL--RNDGY-SV      193
MDLA_PENCA  VRDDIIKELKEVVAQNPNYELVVVGHSLGAAVATLAATDL--RGKGYPSA      192
LIP_RHIDL   VVNDYFPVVQEQLTAHPTYKVIVTGHSLGGAQALLAGMDLYQREPRLSPK      291
LIP_RHIMI   VQNELVATVLDQFKQYPSYKVAVTGHSLGGATALLCALDLYQREEGLSSS      261
            . .. .  .. .    ..*.. .*****.* * *. .. *  *.    .

LIPASE3     ELYTY--GCPRIGNYALAEHITSQGSGANFRVTHLNDIVPRVPPMDFGFS      241
MDLA_PENCA  KLYAY--ASPRVGNAALAKYITAQGN--NFRFTHTNDPVPKLPLLSMGYV      238
LIP_RHIDL   NLSIFTVGGPRVGNPTFAYYVESTGIPFQ-RTVHKRDIVPHVPPQSFGFL      340
LIP_RHIMI   NLFLYTGQPRVGDPAFANYVVSTGIPYR-RTVNERDIVPHLPPAAFGFL      310
            .* .  . .**.*..... * .... *   . *  .. .* **..* ..*.

LIPASE3     QPSPEYWITSGNGASVTASDIEVIEGINSTAGNAGEATVSVV---AHLWY      288
MDLA_PENCA  HVSPEYWITSPNNATVSTSDIKVIDGDVSFDGNTGTGLPLLTDFEAHIWY      288
LIP_RHIDL   HPGVESWIKSGTSN-VQICTSEIE------TKDCSNSIVPFTSILDHLSY      383
LIP_RHIMI   HAGEEYWITDNSPETVQVCTSDLE------TSDCSNSIVPFTSVLDHLSY      354
            . . * **.. . . *  .. ..    . . ...   . .*. *

LIPASE3     FFAISECL--------L    297
MDLA_PENCA  FVQVDAGKGPGLPFKRV   305
LIP_RHIDL   F-DINEGSC-------L   392
LIP_RHIMI   F-GINTGLC-------T   363
            *   ...
```

The above alignment shows that lipase 3 is homologous to the knovit lipase sequences but that the homology is not very high. Deletions or insertions in the lipase 3 sequence was not observed when comparing the sequence with these three lipases. This strengthens the probability that the putative introns have been identified correctly.

A search in SWISS-PROT release 31 database was performed and it did not lead to further sequences with higher homology than that to the above known lipases (Table 5.3).

The sequence with highest homology is a mono- diacyl lipase from *Penicillium camembertii* where the identity is found to 42 %. However the C- terminal of lipase 3 resembles the 2 lipases from Zygomycetes (*Rhizopus* and *Rhizomucor*) and not the *P. camembertii* enzyme.

## Table 5.3. Alignment of coding sequences of the *lipA* gene and gene coding for mono-diacyl lipase from *Penicillium camemberti*

```
LIPASE3    - MFSGRFGVLLTALAALGAAAPAPLAVRSVSTSTLDELQLFAQWSAAAYCS -50
             |    |  |  |  |.||  |'|  |   |  ||||.||     |  || |
MDLA_PENCA- MRLSFFTAL-SAVASLGYALPGKLQSRDVSTSELDQFEFWVQYAAASYYE -49


LIPASE3    - NNIDSK-DSNLTCTANACPSVEEASTTMLLEFDLTNDFGGTAGFLAADNT -99
               |.|      ||.||       |        |          |||   |.| |
MDLA_PENCA- ADYTAQVGDKLSCSKGNCPEVEATGATVSYDFS-DSTITDTAGYIAVDHT -98


LIPASE3    - NKRLVVAFRGSSTIENWIANLDFILEDNDDLCTGCKVHTGFWKAWESAAD -149
             |  .  |  ||||||    .||.|   |    |  ||  ||     |||   |    .|
MDLA_PENCA- NSAVVLAFRGSYSVRNWVADATFV-HTNPGLCDGCLAELGFWSSWKLVRD -147


LIPASE3    - ELTSKIKSAMSTYSGYTLYFTGHSLGGALATLGATVLRNDGY-SVELYTY -198
             |..      -  | |   |||||  |  |||  ||  ||   || |..||.|
MDLA_PENCA- DIIKELKEVVAQNPNYELVVVGHSLGAAVATLAATDLRGKGYPSAKLYAY -197


LIPASE3    - GCPRIGNYALAEHITSQGSGANFRVTHLNDIVPRVPPMDFGFSQPSPEYW -248
             ||  ||  |||  '||  ||   |||  ||  ||  ||   |     |     .|||||
MDLA_PENCA- ASPRVGNAALAKYITAQGN--NFRFTHTNDPVPKLPLLSMGYVHVSPEYW -245


LIPASE3    - ITSGNGASVTASDIEVIEGINSTAGNAGEATVSVV---AHLWYFFAISEC -295
             |||  |  |  | ||| ||  |.  |  |  || |            ||  |||
MDLA_PENCA- ITSPNNATVSTSDIKVIDGDVSFDGNTGTGLPLLTDFEAHIWYFVQVDAG -295
```

```
LIPASE3    - L-------L -297


MDLA_PENCA- KGPGLPFKRV -305


Identity: 126 amino acids (42.42%)
```

The N-terminal of the mature lipase has been determined by N-terminal sequencing to be the serine residue No. 28 of the lipase 3 precursor (SEQ ID NO:9) as shown in Table 5.4 below. Hence the amino acids No. 1 to No. 27 is the signal sequence.

## Table 5.4: Amino acid sequence of the precursor of lipase 3

```
                    5        10       15       20       25       30
                    |        |        |        |        |        |
    1 M F S G R F G V L L T A L A A L G A A A P A P L A V R S V S
   31 T S T L D E L Q L F A Q W S A A A Y C S N N I D S K D S N L
   61 T C T A N A C P S V E E A S T T M L L E F D L T N D F G G T
   91 A G F L A A D N T N K R L V V A F R G S S T I E N W I A N L
  121 D F I L E D N D D L C T G C K V H T G F W K A W E S A A D E
  151 L T S K I K S A M S T Y S G Y T L Y F T G H S L G G A L A T
  181 L G A T V L R N D G Y S V E L Y T Y G C P R I G N Y A L A E
  211 H I T S Q G S G A N F R V T H L N D I V P R V P P M D F G F
  241 S Q P S P E Y W I T S G N G A S V T A S D I E V I E G I N S
  271 T A G N A G E A T V S V V A H L W Y F F A I S E C L L
```

Number of residues : 297.

Residues 167-176 are recognised as a common motif for the serine lipases (PROSITE). The crystal structure for the *Rhizomucor miehei* serine lipase has been examined and the residues in the active site identified (Brady et al., Nature, 1990, **343**:767-770; Derewanda et al., J. Mol. Biol., 1992, **227**:818-839). The active site residues of *R. miehei* lipase have all been conserved in all the lipases and correspond to the following residues in lipase 3: serine 173, aspartic acid 228 and histidine 285.

Lipase 3 contains 7 cysteine residues. Four of these are conserved in the *P. camembertii* lipase where they form. disulphide bonds (Isobe and Nokuhara, Gene, 1991, **103**:61-67). This corresponds to disulphide bonds between residue 62- 67 and 131-134. In addition, two cysteine residues are homologous to two C residues which forms an additional disulphide bond in *Rhizopus* and *Rhizomucor* lipases corresponding to residues 49-295.

Two putative N-glycosylation sites were found in lipase 3 in position 59 and 269. Neither of these are conserved in the other fungal lipases.

EXAMPLE 6

Transformation of *Aspergillus tubigensis* and overexpression of lipase 3 in *A. tubigensis*

The protocol for transformation was based on the teachings of Buxton et al. (Gene, 1985, **37**:207-214), Daboussi et al (Curr. Genet., 1989, **15**: 453-456) and Punt and van den Hondel, (Meth. Enzym., 1992, **216**:447-457).

A multicopy *lip*A strain was produced by transforming the pLIP4 plasmid into *Aspergillus tubigensis* strain 6M 179 using cotransformation with a hygromycin resistant marker plasmid.

A screening procedure used to visualise fungal lipase after ultrathin layer isoelectric focusing was adapted to screen *Aspergillus tubigensis* transformants grown on agar plates. Screening of lipase producers on agar plates was done using 2% olive oil as the substrate for the enzyme (lipase) as well as the inducer for the lipase promoter. In addition, the plates contained a fluorescent dye, Rhodamine B. In the presence of olive oil, the transformants will be induced to secrete lipase. The lipase secreted into the agar plate will hydrolyse the olive oil causing the formation of orange fluorescent colonies that is visible upon UV radiation (350 nm). The appearence of fluorescent colonies was generally monitored after 24 hours of growth. After several days of growth, the lipase producing strains could be identified as orange fluorescent strains that are visible by eye. Under this plate screening condition, the untransformed strain gave no background fluorescence and appeared as opaque pink colonies.

Sixteen transformants that showed orange fluorescent halos were cultivated for 8 days in shake flasks containing 100 ml of minimal medium supplemented with 1% olive oil, 0.5% yeast extract and 0.2% casamino acids. The amount of lipase secreted was quantified by applying 10 $\mu$l of cell-free culture supernatant into holes punched in olive oil-Rhodamine B agar plates and incubating the plates overnight at 37°C. Five transformants with higher lipase production were found.

The cell-free culture supernatants from the five transformants were desalted using NAP 5 columns (Pharmacia) and equilibrated in 1M ammonium sulfate (50 mM sodium acetate, pH 5.5). The desalted culture supernatants were fractionated by hydrophobic interaction chromatography (HIC) on a Biogel Phenyl-5 PW column (Biorad). Elution was done by a descending salt gradient of 1M to 0 M ammonium sulfate (20 mM sodium acetate, pH 5.5). A single discrete protein peak was observed after fractionation. The area of the protein peaks were calculated among the different transformants and compared with the untransformed strain. The best transformant showed a 62-fold increase in the amount of lipase after HIC fractionation.

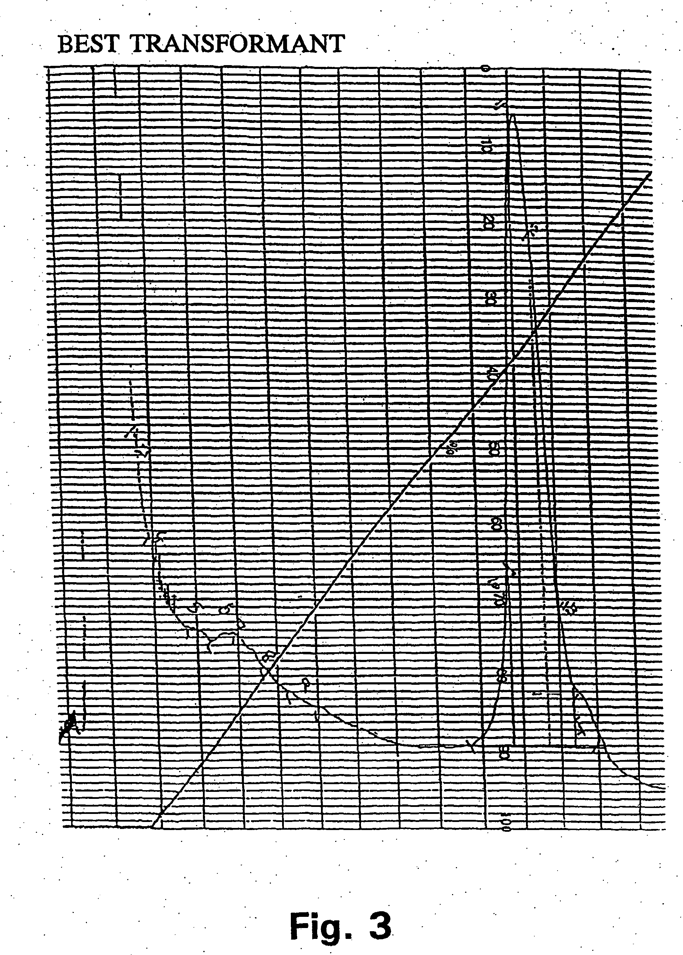

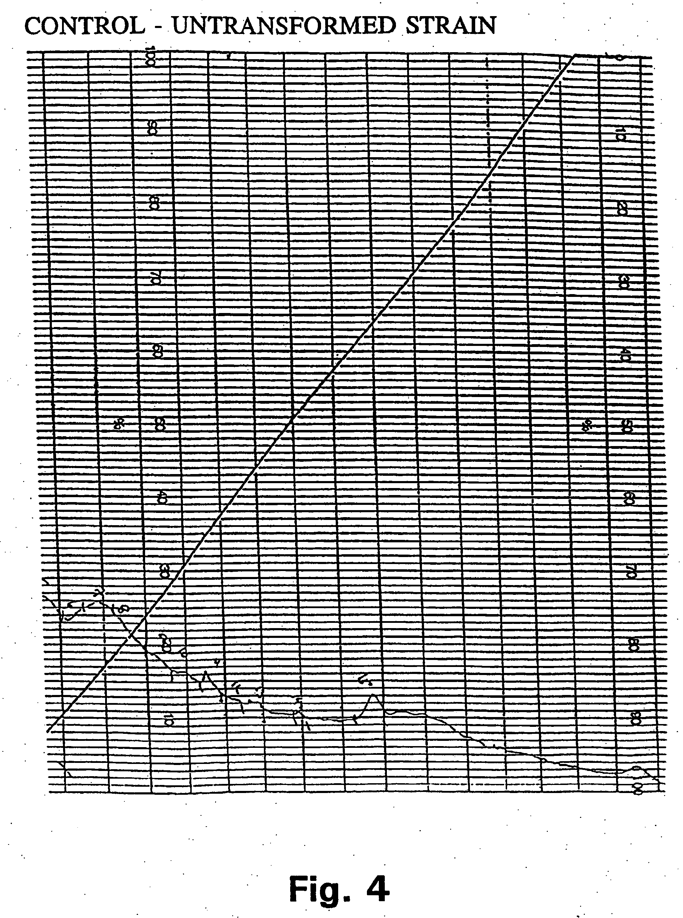

A chromatogram of the HIC fractionated culture supernatant of this transformant is shown in Fig. 3 and a similar chromatogram for the untransformed strain is shown in Fig. 4.

The fraction containing the transformed lipase was freeze-dried. The transformed lipase was carboxymethylated and subjected to N-terminal amino acid sequencing of the first 15 amino acids and it was found that the sequence of the recombinant lipase was exactly the same as the native lipase indicating correct signal sequence cleavage.

The different lipase fractions collected after HIC were separated on a 12% Tris-Glycine SDS gel and silver staining revealed one protein band, confirming the homogeneity of the fractions. In addition, the crude extract showed a major lipase band as the only band that accumulated in the culture supernatant in very high amounts when the fungus was cultured in the olive oil-containing medium.

The recombinant lipase was analysed by matrix-assisted laser-desorption ionisation (MALDI) by means of a time-of-flight (TOF) mass spectrometer as described hereinbefore. The molecular weight of the recombinant lipase was 32,237 Da.

Detection of N-linked oligosaccharides was achieved by digestion of the lipase with endo-$\beta$-N-acetyl-glucosamidase H from *streptomyces* (Sigma). Digestion of recombinant lipase secreted into the growth medium altered the mobility of the band seen on SDS-PAGE which moved as a single band with a molecular mass of about 30 kDa.

Deglycosylated recombinant lipase generated by digestion with endoglycosidase and analysed directly by MALDI mass spectrometry gave a molecular weight of the polypeptide backbone of 2.9, 325 Da.

EXAMPLE 7

Construction of lipase 3 glycosylation mutants

Overexpression of lipase 3 in the *A. tubigensis* strain 6M 179 (Example 6) resulted in overglycosylation of the protein and subsequent reduction of enzyme activity.

In order to circumvent the problem of overglycosylation and loss of activity, several mutated *lipA* genes were, constructed. A molecular model for the three dimensional structure of lipase 3 based on database comparison, with known lipases and their solved crystal structures revealed the surface topology of the two putative-N-glycosylation sites in lipase. The two possible sites responsible for glycosylation are the asparagine residues at N59 and N269. The 2 asparagine residues were changed by mutation either to a threonine residue (T) or to a glutamine residue (Q) . Mutation to threonine (N>T) eliminates the amide group through which asparagine is glycosylated without altering the size of the side chain as well as retaining the polar oxygen. Mutation to glutamine (N>Q) results in an extra carbon at the side chain and retention of the amide group. Three single mutants, N59T; N269T; N269Q and two double mutants, N59TN269T and N59TN269Q, were constructed as it is described in the following.

Mutagenic primers designed to incorporate specific sequence mutations were phosphorylated using T4 polynucleotide kinase as described in the Bio-Rad M13 In Vitro Mutagenesis Kit Manual. Annealing to the single stranded DNA template took place in 20mM Tris, 50mM NaCl, 2mM MgCl$_2$ at a molar ratio of primer to template of 30:1, incubation at 30°C for about 1.5 hours. The synthesis of the second strand was accomplished by T7 DNA polymerase (0.5 units) in a reaction mixture containing 0.4 mM of each dNTP, 0.75 mM ATP, 17.5 mM Tris-Cl (pH 7.4), 3.75 mM MgCl$_2$, 21.5 mM DDT and 4T DNA ligase (5 units) for ligation of the newly synthesized strand to the 5' end of the primers. The reaction was incubated on ice for 5 minutes, then at 25°C for 5 minutes and finally at 37°C for 30 minutes and was stopped by the addition of stop buffer (10 mM Tris pH 8, 10 mM EDTA) and freezing. Reactions were analyzed on a 1% agarose gel in TAE buffer before transformation into-SURE *E.coli* cells. The transformants were analyzed by DNA sequencing.

The SURE *E.coli* transformants containing the mutated *lipA* genes were deposited in accordance with the Budapest Treaty with The National Collections of Industrial and Marine Bacteria Limited (NCIMB) at 23 St. Machar Drive, Aberdeen, Scotland, United Kingdom, AB2 1RY on 24 March 1998 under the following accession numbers. The three single mutants, N59T: NCIMB 40931; N269T: NCIMB 40932; N269Q: NCIMB 40933 and the two double mutants, N59TN269T: NCIMB 40934; N59TN269Q: NCIMB 40935.

EXAMPLE 8

Transformation of *Aspergillus tubigensis* strain 3M *pyrA* with *lipA* mutants encoding lipase 3 glycosylation mutants and expression of the mutated genes

**(i) transformation procedure**

Spores from the *A. tubigensis* strain 3M *pyr A* were cultivated overnight at 34°C in a shake flask containing minimal media supplemented with 2% glucose and 10mM uridine. The mycelia was harvested and resuspended in lysis buffer plus lysing, enzyme. The protoplasts produced were mixed with plasmids encoding the different lipase glycosylation mutants by the co-transformation method.

**(ii) Expression of *lip*A genes coding for mutant lipases**

The transformants were screened for production of mutant lipases. Pure cultures of transformants were propagated in liquid media in the following manner: spores were added at a density of $10^6$/ml and grown at 34°C for 5 days under shaking at 200 rpm. The medium for cultivation of transformants contained 100 ml minimal medium supplemented with 2% sunflower oil (inducer), 1.5% peptone, 0.2 casamino acids and 50 $\mu$g/ml ampicillin. Culture filtrate was collected each day and tested on olive oil-rhodamine plates for lipase activity. The oil-rhodamine plates were inspected for fluorescent halos (lipase activity) after an overnight incubation at 37°C. In addition, a chromogenic assay using 1,2-O-dilauryl-rac-glycero-3-glutaric acid-resorufin ester (Lipase chromogenic substrate, Boehringer Mannheim Biochemica, cat. No. 1179934) was used to double check the filtrates collected on days 2, 3, 4 and 5. Table 8.1 below shows lipase activity of selected trans formants expressing different modified *lip*A genes coding for mutant lipase 3. Lipase activity was measured on day 5 using the above chromogenic assay.

Table 8.1. Lipase activity of lipase 3 enzymes encoded by glycosylation mutants of the *lip*A gene

| Transformant | Lipase type | Lipase activity |
|---|---|---|
| 160 | N59T | 0.478 |
| 161 | N59T | 1.327 |
| S2-2 | N269Q | 0.840 |
| S2-5 | N269Q | 0.102 |
| S2-6 | N269Q | 0.916 |
| S2-7 | N269Q | 0.127 |
| S2-8 | N269Q | 0.100 |
| S3-1 | N269T | 1.048 |
| S3-3 | N269T | 0,145 |
| S3-4 | N269T | 2.86 |
| S3-5 | N269T | 0.068 |
| S3-6 | N269T | 0.086 |
| S3-8 | N269T | 0.254 |
| S4=1 | N59TN269Q | 1.088 |
| S4-2 | N59TN269Q | 0.948 |
| S4-3 | N59TN269Q | 1.75 |
| S4-5 | N59TN269Q | 0.358 |
| S4-7 | N59TN269Q | 0.097 |
| 24-6M | N59TN269T | 0.424 |
| 93-10M | N59TN269T | 0.249 |
| 97-10M | N59TN269T | 0.263 |

Table continued

| Transformant | Lipase type | Lipase activity |
|---|---|---|
| 720-4 3M | N59TN269T | 0.479 |
| Strain 6M 179 | overglycosylated | 0.688 |
| *A. tubigensis* 3M pyr A | untransformed | 0.053 |

In addition, lipase activity in the culture filtrates of transformants S3-4, S4-3 and strain 6M 179 (expressing overglycosylated lipase 3) showed increasing lipolytic activities (Table 8.2) during fermentation from day 2 to day 5.

Table 8.2. Lipase activity in culture filtrates during fermentation (chromogenic assay measured at 572 nm)

| Strain | Day 2 | Day 3 | Day 4 | Day5 |
|---|---|---|---|---|
| S3-4 | 0.592 | 0.985 | 1.276 | 2.86 |
| S4-3 | 0.176 | 0.452 | 0.680 | 1.75 |
| 6M 179 | | 0.443 | 0.672 | 0.688 |

EXAMPLE 9

Fermentation and purification of wild-type and modified lipase 3

**(i) Fermentation**

Spores ($10^6$/ml) of *Aspergillus tubigensis* strains expressing different lipase 3 glycosylation mutants were used to inoculate shake flasks containing 200 ml of minimal medium supplemented with 2% sunflower oil (inducer), 1.5% peptone, 0.2% casamino acids and 50 $\mu$g/ml ampicillin. The cultures were grown with shaking at 200 rpm for 4 days at 34°C. The culture filtrate was separated from the mycelia by filtration through nylon gauge.

**(ii) Purification of lipase**

Samples of culture supernatant of S2-6, S3-4, S4-1 and S4-3 fermentations, respectively were all treated in the same manner. A 15 ml sample was first desalted on a PD-10 column equilibrated in 20 mM triethanolamine, pH 7.3. The desalted sample was applied to a Source Q15 (HR5/5) anion exchanger equilibrated in 20 mM triethanolamine, pH 7.3. Flow 1.5 m ml/min. The column was washed with equilibration buffer until a stable baseline was obtained. Lipase activity was then eluted with a 30 ml linear gradient of from 0 to 0.53 M NaCl in equilibration buffer. Fractions of 1.5 ml were collected. The fractions were screened for activity using the plate assay on tributyrin as described above.

To the pool of lipase activity from Source Q15 was added ammonium sulfate to 1 M and the sample was applied to a Source Phenyl HIC (HR5/5) column equilibrated in 20 mM sodium acetate (pH 5.5), 1 M ammonium sulfate. The column was washed with equilibration buffer. Lipase was eluted with a linear gradient from 1 to 0 M ammonium sulfate in 20 mM sodium acetate (pH5.5), flow 1.5 ml/min. Fractions of 0.75 ml were collected. The lipase peak fractions were totally homogeneous (one band) as examined by SDS-PAGE followed by silver staining as described above.

The four different lipase glycosylation mutants behaved the same way throughout the purification.

**(iii) Determination of specific activity**

The specific activity was determined for the four purified lipase mutants. Both with respect to activity on tributyrin (LUT) as described above and with respect to sun flower oil (LUS) as also described previously. Protein was determined as described as above. The results are summarized in Table 9.1

Table 9.1. Specific activity of mutant lipases as compared to wild-type lipase 3 and overglycosylated lipase 3.

| | Lip. 3 Wild-type | Lip. 3 over glycosylated | S2-6 | S3-4 | S4-1 | S4-3 |
|---|---|---|---|---|---|---|
| Protein $\mu$g/ml | 7.6 | 10.5 | 27.2 | 24.1 | 25.2 | 26.3 |
| LUT/ml | 30 | 37 | 86 | 54 | 73.6 | 72.6 |

Table continued

|  | Lip. 3 Wild-type | Lip. 3 over glycosylated | S2-6 | S3-4 | S4-1 | S4-3 |
|---|---|---|---|---|---|---|
| LUT/mg protein | 3,947 | 3,524 | 3,162 | 2,241 | 2,921 | 2,756 |
| LUS/ml | 104 | 55 | 283 | 88.2 | 261 | 248 |
| LUS/mg protein | 13,684 | 5,238 | 10,404 | 3,660 | 10,357 | 9,415 |

It can be seen that the specific activity of the mutant lipases expressed in S2-6, S4-1 and S4-3 is twice the specific activity of the overglycosylated lipase 3 when measured (LUS) on sunflower oil which contains long chain fatty acids like lipids normally present in flour and dough. When measured on tributyrin (LUT) the differences in specific activity are less pronounced. This could be explained by the fact that a possible "shadowing" of the active site by overglycosylation will be less detrimental if the substrate is small like tributyrin as opposed to the long fatty acid chains in sunflower oil. The specific activity of the S3-4 lipase 3 is lower than that of overglycosylated lipase 3 (measured on sunflower oil).

When assayed in the LUS assay none of the mutants have quite as good specific activity as the wild-type lipase 3, but as shown in a following example, the enzymatic activity is higher for the glycosylation mutants than for the wild-type lipase 3 in a dough system.

### (iv) Determination of molecular weight

The apparent molecular weight of the purified mutant lipases was determined by running the lipases on one SDS-PAGE gel. The results are shown in table 9.2.

Table 9.2. Molecular weight of wild-type, overglycosylated and mutant lipases

|  | Lip. 3 Wild-type | Lip. 3 Overglycosylated | S2-6 | S3-4 | S4-1 | S4-3 | S5 |
|---|---|---|---|---|---|---|---|
| MW (kDa) | 34.8 | 38.9 | 34.8 | 34.8 | 31.2 | 31.2 | 31.2 |

It is seen that the overglycosylated lipase gives rise to a lipase 3 molecule which has a larger molecular weight than the wild-type native lipase 3. By removing one of the two potential N-glycosylation sites as it is done in S2-6 (N269Q) and, in 3S-4 (N269T) a lower molecular weight was obtained, but it is still slightly higher than for the wild-type lipase 3, indicating overglycosylation at the second glycosylation site. By removing both potential N-glycosylation sites as in S4-1 and S4-3 (both N59T N269Q), a molecular weight clearly lower than that of the wild-type lipase 3 is obtained which is in agreement with a total lack of N-glycosylation in this mutant.

### (v) Determination of the isoelectric point for lipase mutants

The isoelectric points for the 4 different lipase glycosylation mutants were determined by chromatofocusing on a Mono P (HR5/5) to be pH 4.1 for all the mutant enzymes. The column was equilibrated in 25 mM bib-tris, pH 6.10. A pH. gradient was generated by running 100% of a 10% polybuffer 74 in water (pH 3.8 with HCl) plus 2.5% betaine, this eluted the lipase(s). Flow 0.70 ml/min. Fractions of 0.4 ml were collected.

### (vi) Determination of temperature stability of mutant lipase S4-1

A sample of purified lipase S4-1 (296 LUT/ml) was diluted 1:1 with 200 mM NaAc, pH 5.0. Eppendorf tubes with 1 ml of this diluted sample were incubated for 1 hour in a waterbath at 30, 40, 50 and 60°C respectively. A control was treated the same way but left at room temperature. The remaining lipase activity was determined by the p-nitrophenyl acetate assay as described previously. The results are shown below in Table 9.3.

Table 9.3. Temperature stability of mutant lipase (S4-1) compared to wild-type lipase 3

| Temperature (°C) | Mutant lipase S4-1 (activity) |
|---|---|
| 20 (Control) | 100% |
| 30 | 107% |
| 40 | 96% |
| 50 | 89% |

Table continued

| Temperature (°C) | Mutant lipase S4-1 (activity) |
|---|---|
| 60 | 53% |

**(vii) Determination of pH stability of mutant lipase S3-4**

Samples of 200 μl purified mutant lipase S3-4 were added to 5 ml of 50 mM buffer (pH 3.5, 4.0, 5.0 NaAc and pH 6.0, 7.0, 8.0 phospate buffer). The control was diluted in to 5.0 ml of 4 mM NaAc, pH 5.5. After 4 days at 20°C the samples were measured for residual activity by the Modified Food Chemical Codex assay for lipase activity as described above. The mutant lipase S3-4 was very stable in the pH range from 4.0 to 8.0 where it maintained about 100% activity relative to the control. At pH 3.5 the lipase maintained 88% activity as compared to the control. The results are shown in Table 9.4.

Table 9.4. pH stability of mutant lipase S3-4

| pH | Activity (LUT/ml) | Activity (%) |
|---|---|---|
| Control (pH 5.5) | 30.6 | 100 |
| 3.5 | 26.9 | 88 |
| 4.0 | 29.3 | 96 |
| 5.0 | 30.3. | 99 |
| 6.0 | 35.3 | 115 |
| 7.0 | 33.3 | 109 |
| 8.0 | 31.3 | 102 |

EXAMPLE 10

Baking experiments using lipase 3

10.1. Baking procedures and analytical methods

**(i) Baking procedure for Danish toast bread**

Flour (Danish reform flour) 2000 g, dry yeast 30 g, salt 30 g and water corresponding to 400 Brabender units + 3%, was kneaded in a Hobart Mixer with hook for 2 min. at low speed and 10 min. at high speed.. Dough temperature after kneading was 25 °C. Resting time was 10 min. at 30°C. The dough was scaled 750 g per dough and rested again for 5 min at 33°C and 85% RH. After moulding on a Glimik moulder, the dough were proofed in tins for 50 min at 33°C and baked in a Wachtel oven for 40 min at 220°C with steam injection for 16 sec. After cooling, the bread was scaled and the volume of the bread was measured by the rape seed displacement method. The specific volume is calculated by dividing the bread volume (ml) by the weight (g) of the bread.
The crumb was evaluated subjectively using a scale from 1 to 5 where 1 = coarsely inhomogeneous and 5 - nicely homogeneous.
Three breads baked in tins with lid were stored at 20°C and used for firmness measurements and pore measurements by means of an Image Analyzer.

**(ii) Baking procedure for Danish rolls**

Flour (Danish reform) 1500 g, compressed yeast 90 g, sugar 24 g, salt 24 g and water corresponding to 400 Brabender units -2% were kneaded in a Hobart mixer with hook for 2 min. at low speed and 9 min at high speed. After kneading, the dough temperature was 26°C. The dough was scaled 1350 g. After resting for 10 min. at 30°C, the dough was moulded on a Fortuna moulder after which the dough was proofed for 45 min. at 34°C and baked in a Bago oven for 18 min. at 220°C with steam injection for 12 sec. After cooling, the rolls were scaled and the volume of the rolls was measured by the rape seed displacement method. Specific volume is calculated as described above.

**(iii) Determination of pore homogeneity**

The pore homogeneity of the bread was measured by means of an image analyzer composed of a standard CCD-video camera, a video digitiser and a personal computer with WinGrain software. For every bread, the results of pore diameter in mm and pore homogeneity were calculated as an average of measurements from 10 slices, of bread. The pore homogeneity was expressed in % of pores that are larger than 0.5 times the average of pore diameter and smaller than 2 times the average diameter.

**(iv) Determination of firmness**

The firmness of bread, expressed as $N/dm^2$, was measured by means of an Instron UTM model 4301 connected to a personal computer. The conditions for measurement of bread firmness were:

| | |
|---|---|
| Load Cell | Max. 100 N |
| Piston diameter | 50 mm |
| Cross head speed | 200 mm/min |
| Compression | 25% |
| Thickness of bread slice | 11 mm |

The result was an average of measurements on 10 bread slices for every bread.

**(v) Determination of gluten index**

Gluten index was measured by means of a Glutomatic 2200 from Perten Instruments (Sweden). Immediately after proofing, 15 g of dough was scaled and placed in the Glutomatic and washed with 500 ml 2% NaCl solution for 10 min. The washed dough was transferred to a Gluten Index Centrifuge 2015 and the two gluten fractions were scaled and the gluten index calculated according to the following equation:

```
Gluten index = (weight of gluten remaining on the sieve x
100)/total weight of gluten
```

**(vi) Extraction of lipids from dough**

30 g of fully proofed dough was immediately frozen and freeze-dried. The freeze-dried dough was milled in a coffee mill and passed through a 235 $\mu$m screen. 4 g freeze-dried dough was scaled in a 50 ml centrifuge tube with screw lid and 20 ml water saturated n-butanol (WSB) was added. The centrifuge tube was placed in a water bath at a temperature of 100°C for 10 min. after which the tubes were placed in a Rotamix and turned at 45 rpm for 20 min. at ambient temperature. The tubes were again placed in the water bath for 10 min. and turned on the Rotamix for another 30 min. at ambient temperature.
The tubes were centrifuged at 10,000 x g for 5 min. 10 ml of the supernatant was pipetted into a vial and evaporated to dryness under nitrogen cover. This sample was used for HPLC analysis.
A similar sample was fractionated on a Bond Elut Si (Varian 1211-3036). The non-polar fraction was eluted with 10 ml cyclohexan:isopropanol:acetic acid (55:45:1) and evaporated to dryness. This sample was used for GLC analysis.

**(vii) HPLC analysis**

Column: LiChrospher 100 DIOL 5 $\mu$m (Merck art. 16152) 250x4 mm with a water jacket of a temperature of 50°C.
Mobile phases:

A: heptan:isopropanol:n-butanol:tetrahydrofuran:isooctan:water (64.5:17.5:7:5:5:1)

B: isopropanol:n-butanol:tetrahydrofuran:isooctan:water (73:7:5:5:10)

The mobile phases contained 1 mmol trifluoroacetic acid per 1 mobile phase and were adjusted to pH 6.6 with ammonia.
Pump: Waters 510 equipped with a gradient controller.
Gradient:

| Flow (ml/min) | Time (min) | A (%) | B (%) |
|---|---|---|---|
| 1.0 | 0 | 100 | 0 |
| 1.0 | 25 | 0 | 100 |
| 1.0 | 30 | 0 | 100 |
| 1.0 | 35 | 100 | 0 |
| 1.0 | 40 | 100 | 0 |

Detector: CUNOW DDL21 (evaporative light-scattering); temperature 100° C; voltage: 600 volt; air flow: 6.0 l/min.
Injector: Hewlett Packard 1050; injection volume: 50 $\mu$l.
The samples for analysis were dissolved in 5 ml chloroform:methanol (75:25), sonicated for 10 min and filtered through a 0.45 $\mu$m filter.

**(viii) GLC analysis**

Perkin Elmer 8420 Capillary Gas Chromatograph equipped with WCOT fused silica column 12.5 m x 0.25 mm coated with 0;1 $\mu$m stationary phase of 5% phenyl-methyl-silicone (CP Sil 8 CB from Crompack) .
Carrier: Helium....
Injection: 1.5 $\mu$l with split
Detector: FID 385°C

| Oven program: | 1 | 2 | 3 | 4. |
|---|---|---|---|---|
| Oven temperature, °C | 80 | 200 | 240 | 360 |
| Isothermal time, min | 2 | 0 | 0 | 10 |
| Temperature rate, °C/min | 20 | 10 | 12 | |

Sample preparation: 50 mg non-polar fraction of wheat lipids was dissolved in 12 ml heptane:pyridine (2:1) containing 2 mg/ml heptadecane as internal standard. 500 $\mu$l of the solution was transferred to a crimp vial and 100 $\mu$l N-methyl-N-trimethylsilyl-trifluoracetamide was added. The mixture was allowed to react for 15 min at 90°C.
Calculation: Response factors for mono-, di- and triglycerides and free fatty acids were determined from reference mixtures of these components. Based on these response factors, the glycerides and the free fatty acids were calculated in wheat lipids.

10.2. Baking experiments with lipase 3 in Danish toast bread

The effect of adding lipase 3 to a dough for making Danish toast bread was evaluated. The enzyme was added as a freeze-dried preparation on maltodextrin together with the other ingredients. The results of the baking tests are shown in Tables 10.1 to 10.4.

Table 10.1

| Lipase LUS/kg flour | 0 | 5,000 | 15,000 | 25,000 |
|---|---|---|---|---|
| Specific volume of bread | 4.43 | 4.43 | 4.22 | 4.37 |
| Firmness Day 1 | 35 | 33 | 32 | 30 |
| Firmness Day 7 | 90 | 90 | 85 | 73 |

Table 10.2

| Lipase LUS/kg flour | 0 | 5,000 | 15,000 | 25,000 |
|---|---|---|---|---|
| Average diameter of the crumb pore, mm | 2.96 | 2.33 | 2.47 | 2.65 |

Table continued

| Lipase LUS/kg flour | 0 | 5,000 | 15,000 | 25,000 |
|---|---|---|---|---|
| Homogeneity of crumb pore, % | 64.9 | 73.8 | 66.0 | 67.1 |
| Porosity, % | 85.9 | 84.7 | 85.5 | 85.1 |
| Gluten index, % | 42 | 45.5 | 55 | 65 |

Table 10.3

| Lipase LUS/kg flour | 0 | 5,000 | 15,000 | 25,000 |
|---|---|---|---|---|
| Fatty acids, % | 0.090 | 0.148 | 0.218 | 0.241 |
| Monoglycerides, % | 0.017 | 0.031 | 0.035 | 0.039 |
| Diglycerides, % | 0.020 | 0.036 | 0.040 | 0.045 |
| Triglycerides, % | 0.790 | 0.714 | 0.673 | 0.622 |

Table 10.4

| Lipase LUS/kg flour | 0 | 5,000 | 15,000. | 25,000 |
|---|---|---|---|---|
| Monogalactosyl diglyceride, % | 0.073 | 0.040 | 0.025 | 0.018 |
| Digalactosyl diglyceride, % | 0.244 | 0.220 | 0.182 | 0.127 |
| Digalactosyl monoglyceride, % | 0.008 | 0.022 | 0.044 | 0.054 |
| Phosphatidyl choline, % | 0.064 | 0.073 | 0.055 | 0.041 |
| Lysophosphatidyl choline, % | 0.164 | 0.182 | 0.171 | 0.165 |

By the addition of up to about 5,000 LUS/kg flour of the lipase no change in bread volume was observed, but at a higher dosage of lipase 3 there was a tendency to a small but not statistically significant decrease in volume (Table 10.1).

From the results in Table 10.2 it appears that lipase 3 improved the bread crumb homogeneity and that the average diameter of the crumb pores was reduced significantly.

The gluten index also clearly correlated to the addition of lipase 3 as an indication of a more firm gluten caused by the modification of the wheat lipid components causing better dough stability and a more homogeneous bread pore structure. However, these modifications appeared to be optimal at the addition of 5,000 LUS/kg flour of lipase 3 whereas a higher dosage resulted in a too strong modification of the wheat gluten.

The results of the GLC and HPLC analyses (Table 10.3) clearly demonstrated that the triglycerides in the dough were hydrolysed. But more interestingly, there was also observed a modification of the glycolipids, monogalactosyl diglyceride and digalactosyl diglyceride. These components were converted to the more polar components monogalactosyl monoglyceride and digalactosyl monoglyceride. As digalactosyl monoglyceride is a more surface active component than digalactosyl diglyceride it is assumed that this component contributed to the observed improved crumb cell structure and homogeneity. It also appeared that phospholipids like phosphatidyl choline were only modified to a very small extent.

10.3. Baking experiments with lipase 3 in Danish rolls

The effect of adding lipase 3 to a dough for making Danish rolls was evaluated. The enzyme was added as a freeze-dried preparation on maltodextrin together with the other ingredients. The results of the baking tests are shown in Tables 10.5 to 10.7.

Table 10.5

| Lipase 3 LUS/kg flour | 0 | 10,000 | 20,000 | 30,000 |
|---|---|---|---|---|
| Specific volume of bread (45 min fermentation) | 6.86 | 7.04 | 6.35 | 6.36 |

Table continued

| Lipase 3 LUS/kg flour | 0 | 10,000 | 20,000 | 30,000 |
|---|---|---|---|---|
| Specific volume of bread (65 min fermentation) | 8.30 | 8.59 | 8.23 | 8.04 |
| Subjective evaluation of crumb homogeneity | 3 | 5 | 4 | 4 |

Table 10.6

| Lipase 3 LUS/kg flour | 0 | 10,000 | 20,000 | 30,000 |
|---|---|---|---|---|
| Free fatty acids, % | 0.060 | 0.126 | 0.173 | 0.211 |
| Monoglycerides, % | 0.028 | 0.050 | 0.054 | 0.063 |
| Diglycerides, % | 0.103 | 0.095 | 0.110 | 0.104 |
| Triglycerides, % | 0.705 | 0.561 | 0.472 | 0.436 |

Table 10.7

| Lipase 3 LUS/kg flour | 0 | 5,000 | 15,000 | 25,000 |
|---|---|---|---|---|
| Digalactosyl diglyceride, % | 0.204 | 0.187 | 0.154 | 0.110 |
| Digalactosyl monoglyceride, % | 0.007 | 0.026 | 0.047 | 0.074 |
| Phosphatidyl choline, % | 0.077 | 0.078 | 0.077 | 0.063 |
| Lysophosphatidyl choline, % | 0.153 | 0.161 | 0.162 | 0.150 |

It is apparent from the results shown in Table 10.5 that the addition of lipase 3 does not significantly increase the volume of the rolls. Furthermore, lipase 3 was found to improve the homogeneity of the crumb.
The GLC and HPLC analyses of the wheat lipids, as shown in Tables 10.6 and 10.7, demonstrated the modification of these lipids.

EXAMPLE 11

Comparative baking experiments using lipase 3 and commercial lipase products

Lipase 3 was tested in Danish rolls in a comparative test

with two commercially available lipase products: NOVOZYM 677 BG (no. 1885) from Novo Nordisk (Denmark) and Lipase A "Amano 6" (no. 1757) from Amano (Japan). The results are summarized in Table 11.1.

Table 11.1

| Lipase | Control | Lipase 3 | No. 1885 | No. 1757 | No. 1757 |
|---|---|---|---|---|---|
| LUS/kg flour | 0 | 500 | 500 | 500 | 1,000 |
| Specific volume of rolls | 5.94 | 6.05 | 6.36 | 6.22 | 6.58 |

It is apparent from the results in Table 11.1 that lipase 3 has no effect on the volume of rolls whereas a significant volume effect was observed for the commercial enzyme products no. 1885 and no. 1757.

EXAMPLE 12

Comparative experiments showing the relative hydrolysis of triglycerides compared to hydrolysis of DGDG

Lipase 3, lipase no. 1885 and lipase no. 1757 were tested in Danish rolls with the purpose of studying the relative hydrolysis of triglycerides as compared to the hydrolysis of digalactosyl diglyceride (DGDG). Fully proofed dough was freeze-dried and extracted with water saturated n-butanol. The extracted lipids were analysed by GLC and HPLC as described above. From the analytical results the degree of hydrolysis of triglycerides compared to the degree of hydrolysis of digalactosyl diglyceride (DGDG) at different levels of lipases was calculated. The levels of lipase used were for No. 1885: 0, 400 and 800 LUS/kg flour; for No. 1757: 0, 1770 and 2360 LUS/kg flour and for lipase 3: 0, 10,000 and 20,000 LUS /kg flour. The results are shown in Table 12.1'..

Table 12.1

| Lipase | | | | | |
|---|---|---|---|---|---|
| No. 1885 | | No. 1757 | | Lipase 3 | |
| % triglyceride | % DGDG | % triglyceride | % DGDG | % triglyceride | % DGDG |
| 0 | 0 | 0 | 0 | 0 | 0 |
| 31.8 | 2 | 14.2 | 1.5 | 20.4 | 8.3 |
| 43.6 | 3 | 17.1 | 3.6 | 33 | 24.5 |

From the results in Table 12.1 it is apparent that lipase 3 has a significant effect on the hydrolysis of digalactosyl diglyceride whereas the effect of the two commercial available lipases, No. 1757 and No. 1885, are negligible.

EXAMPLE 13

Baking experiments using lipase 3 in combination with a diacetyl tartaric acid ester of mono- and diglycerides of fatty acids

Lipase 3 was tested in combination with a diacetyl tartaric acid ester of mono- and diglycerides of edible fatty acids having a saponification value of 355 and an acid value of 60 (PANODAN A2020 DATEM). The rolls were baked after four different fermentation times 45, 65, 85 and 105 minutes. Lipase 3 was added to the dough ingredients as a freeze-dried powder. Pore homogeneity was subjectively evaluated on a scale from 1 to 5 where 1 = course inhomogeneous and 5 = nice homogeneous. The results of the experiments are shown in Table 13.1.

Table 13.1

| DATEM, % | 0.35 | 0.15 | 0.25 | 0.35 |
|---|---|---|---|---|
| Lipase 3, LUS/kg flour | 0 | 5,000 | 5,000 | 5,000 |
| Sp. Volume, 45 min. | 4.98 | 4.97 | 5.00 | 5.39, |
| Sp. Volume, 65 min. | 7.00 | 7.44 | 7.33 | 7.88 |
| Sp. Volume, 85 min. | 7.49 | 7.81 | 8.28 | 8.57 |
| Sp. Volume, 105 min. | 8.06 | 7.99 | 8.87 | 8.91 |
| Pore homogeneity, 105 min. | 3 | 5 | 5 | 5 |

It appears from Table 13.1 that lipase 3 in combination with DATEM improved the pore homogeneity and it was found that there was a combined effect which means that it is possible to use DATEM at a much lower concentration in combination with lipase 3 and still obtain the same volume and a better crumb.

EXAMPLE 14

Baking experiment with lipase 3 in the preparation of Danish rolls with and without the addition of soy oil

The effect of purified wild-type lipase 3 was tested in the preparation of Danish rolls with and without the addition of soy oil to the dough. The baked rolls were evaluated for specific volume, crumb pore homogeneity and bread crust quality. Specific volume was measured as previously described. Pore homogeneity was subjectively evaluated on a scale from 1 to 10 where 1 = course inhomogeneous and 10 = nice homogeneous. Bread crust quality was evaluated on a scale from 1 to 10 where 1 = low quality and 10 = high quality.

Fully proofed doughs from these baking tests were frozen and freeze-dried. The freeze-dried doughs were extracted with water saturated butanol and the content of free fatty acids was determined by GLC analysis as also described in Example 10. The results from the baking tests and analysis of free fatty acids are summarized in Table 14:1.

Table 14.1. Evaluation of roll bread quality and the content of free fatty acid in proofed dough

| Lipase 3 LUT/kg flour | Soy oil in dough % | Specific bread volume cm$^3$/m | Pore homogeneity | Bread crust quality | Free fatty acids %o |
|---|---|---|---|---|---|
| 0 | 2 | 7.18 | 2 | 2 | 1.27 |
| 1,500 | 2 | 6.94 | 3 | 4 | 1.65 |
| 4,500 | 2 | 6.85 | 7 | 6 | 2.10 |
| 13,500 | 2 | 6.38 | 8 | 5 | 2.84 |
| 0 | 0 | 6.60 | 3 | 2 | 1.34 |
| 1,500 | 0 | 6.56 | 3 | 3 | 1.53 |
| 4,500 | 0 | 6.31 | 6 | 6 | 2.04 |
| 13, 500 | 0 | 5.62 | 7 | 6 | 2.71 |

From the baking experiment it appears that, lipase 3 improves both the pore homogeneity and crust quality both in bread with and without added oil. By adding a high level of lipase 3 (13, 500 LUT/kg flour) a reduction in bread volume was observed.

The results of the analysis for free fatty acids in dough from these baking experiments are also shown in Table 14.1. From these results it appears that lipase 3 is active in dough irrespective of whether oil is added or not. Furthermore, the level of free fatty acids in the dough was at the same level in doughs with and without oil.

EXAMPLE 15

Formation of ethyl ester in dough by adding lipase 3

As shown in Example 14, free fatty acids are produced when lipase 3 is added to a dough. During fermentation of a bread dough, the yeast produces carbon dioxide and ethanol, and the level of ethanol in a dough is normally in excess of 1% at the end of proofing. When lipase 3 is present in the dough, this enzyme not only catalyses the hydrolysis of triglycerides, but it was surprisingly found that ethyl ester of fatty acids is also formed.

Ethylesters of fatty acid is a well-known flavour component which is i.a. used to mask off-flavours in fat based food. Baking experiments not reported here have shown that lipase added to a dough is able to mask "old" taste of bread which occurs when bread is made from flour stored at 35°C for 3 months. This might be explained by the formation of ethyl esters in the dough.

The amount of ethyl ester of fatty acids was determined by extracting freeze-dried dough with water saturated butanol. The isolated fat phase was analysed by GLC-MS to determine both the identity and the amount of ethyl esters of fatty acids. The results from these analyses are shown in Table 15.1

Table 15.1. Content of ethyl ester of fatty acids in dough with added lipase

| Lipase 3, LUT/kg flour | Ethyl ester of fatty acids, ppm |
|---|---|
| 0 | 8 |
| 1,500 | 9 |
| 4,500 | 20 |
| 13,500 | 93 |

The results clearly show an increasing level of ethyl ester of fatty acids with increasing dosage of lipase in the dough.

EXAMPLE 16

The effect of lipase 3 in sponge cakes

The effect of lipase 3 was tested in a sponge cake made by the following recipe and procedure.

| | |
|---|---|
| Sugar | 208 g |
| Flour | 188 g |
| Corn starch | 60 g |
| Baking powder | 14 g |
| Egg | 200 g |
| GATODAN 504, sponge cake gel* | 18 g |
| Water | 150 g |
| Lipase 3 | see below |

*Emulsifier consisting of glycerol and propylene glycol esterified with edible fatty acids (28%), ethanol (8%), Grindsted PS 409 (25% sodium stearate in glycerol) (6%) and water (58%).

All the ingredients were mixed for 6 minutes using a Hobart N 50. mixer. The cake mix was scaled 3 x 175 g into sponge cake tins and-baked for 35 minutes at 180°C.

Specific volume of the cake was measured by the rape seed displacement method and the softness of the cake was measured after storage at 20°C for 7 days using an Instron Food Tester.

The results of the baking test are summarized in Table 16.1.

Table 16.1. Specific cake volume and softness after 7 days of storage (hPa)

| Lipase 3 (LUT/kg flour) | Specifik cake volume ($cm^3$/g) | Softness after 7 days of storage (hPa) |
|---|---|---|
| 0 | 6.31 | 59 |
| 1,000 | 6.34 | 47 |
| 2,000 | 6.31 | 41 |
| 4,000 | 6.31 | 41 |
| 10,000 | 6.31 | 41 |

From the results it appears that lipase 3 improves the cake softness without changing the volume of the cake.

EXAMPLE 17

Baking experiment using lipase 3 and optionally hemicellulase for the preparation of rye/wheat bread.

Rye/wheat bread was made from a dough containing the following ingredients: Wheat flour, 667 g; sifted rye flour, 1333 g; "Back aroma sauer" 40 g; compressed yeast, 60 g; salt 44 g; water, 1160 g; lipase 3, see below; GRINDAMYL™ H 121 (commercial hemicellulase product of Grindsted Products, Brabrand, Denmark).

The dough was mixed using a Kemper mixer for 2 min. at low speed and for 11 min. at high speed. Dough temperature 27°C, resting time 30 min. at 32°C, scaling 800 g, proofing 20 min. at 32°C and 85% RH, baking for 30 min. at 230°C and 10 sec. steam.

Dough stickiness was evaluated using a scale from 1 to 5 where 1 = very sticky and 5 = normal not sticky. Specific bread volume was also evaluated. The results are shown in Table 16.1.

Table 17.1. The effect of lipase 3 on stickiness and bread volume with and without addition of GRINDAMYL™ H 121

| GRINDAMYL™ H 121 ppm | Lipase 3 LUT/kg flour | Specific volume $cm^3$/g | Dough stickiness |
|---|---|---|---|
| 150 | 0 | 2,48 | 4 |
| 150 | 10,000 | 2,59 | 5 |

Table continued

| GRINDAMYL™ H 121 ppm | Lipase 3 LUT/kg flour | Specific volume cm³/g | Dough stickiness |
|---|---|---|---|
| 200 | 0 | 2,79 | 4 |
| 200 | 10,000 | 2,74 | 5 |

The addition of Lipase 3 in combination with hemicellulase to rye/wheat bread clearly improved the handling properties of the dough in that the dough was leas sticky.

EXAMPLE 18

Evaluation of the effect in model dough system of lipase 3 produced by A. tubigensis transformants.

Lipases produced by five *A. tubigensis* transformants: 161, S2-6, S3-4, S4-1 and 720-4 3M (see Table 8.1) were tested in a model dough system at different concentrations and the formation of free fatty acid was analyzed according to the following procedure:

A dough was prepared using the following ingredients: flour, 50 g; dry yeast, 0.375 g; NaCl, 0.75 g; water, 28 g, lipase, see below. Flour, dry yeast and salt was mixed for 1 min. in a Brabender mixing bowl (50 gram). Lipase and water was added to the dough followed by mixing for 6 min. at 62 rpm. The dough was transferred to, a beaker provided with a lid and the dough was fermented 60 min. at 32°C. The dough was freeze-dried. The freeze-dried dough was grounded and sieved and extracted with water saturated butanol (WSB). WSB, was evaporated under a stream of $N_2$ and the content of free fatty acids were determined by spectrophotometry according to Kwon, D.Y. and J.S. Rhee, 1986, JAOCS 63:89.

In a first experiment the purified wild-type lipase 3 was compared with the mutant enzyme produced by transformant S3-4 at different concentrations as shown in Table 18.1. The results clearly showed an increased level of fatty acid with increasing level of enzyme dosage. However it was also evident that wild-type lipase 3 levels off at about 3 ‰ fatty acid at high lipase dosage whereas the lipase produced by transformant S3-4 continued to produce more fatty acids at increased enzyme dosage up to about 5 ‰ fatty acid.

Table 18.1. Release of free fatty acids in a dough model system

| LUT/kg | Lipase 3 ‰ fatty acid | Transformant lip 3-4 ‰ fatty acid |
|---|---|---|
| 0 | 0.81 | 0.81 |
| 1,000 | 1.02 | 1.66 |
| 2,500 | 1.35 | 2.38 |
| 5,000 | 2.25 | 4.40 |
| 10,000 | 2.81 | 4.91 |

Lipases produced by transformants 161, S2-6, S4-1 and 720-4 3M were also tested by the same procedure with results as shown in table 18.2.

Table 18.2. Release of free fatty acids in a dough model system

| LUT/kg flour | 161 ‰ fatty acid | S2-6 ‰ fatty acid | S4-1 ‰ fatty acid | 720-4 3M ‰ fatty acid |
|---|---|---|---|---|
| 0 | 1.57 | 1.12 | 1.12 | 1.57 |
| 1,000 | 2.03 | 2.05 | 2.39 | 2.18 |
| 2,500 | 3.46 | 2.58 | 2.91 | 2.85 |
| 5,000 | 3.55 | 4.35 | 3.92 | 3.89 |
| 10,000 | 5.29 | 6.15 | 6.21 | 5.95 |

The results clearly demonstrated that the five different transformant lipases were more active in a dough as compared to wild-type lipase 3 when compared at the same enzyme dosage (LUT/kg).

EXAMPLE 19

Baking experiment using lipases produced by S3-4 and S4-1 in the preparation of Danish rolls.

Baking experiments where Danish rolls were made with different dosages of lipase produced by transformant S3-4 and S4-1, respectively, was carried out. After baking, bread specific volume was determined and the crumb homogeneity was evaluated subjectively as described above. The results from the baking tests are shown in table 19.1.

Table 19.1. The effect on bread quality of lipases produced by transformants S3-4 and S4-1, respectively

| Enzyme producing transformant | Dosage LUT/kg flour | Specific volume cm$^3$/g | Crumb score |
| --- | --- | --- | --- |
| | 0 | 6;52 | 4 |
| S3-4 | 1,000 | 6,64 | 4 |
| S3-4 | 2,500 | 6,61 | 5 |
| S3-4 | 7,500 | 5,88 | 8 |
| S4-1 | 1,000 | 6,08 | 5 |
| S4-1 | 2,500 | 5,9 | 7 |
| S4-1 | 5,000 | 5,51 | 9 |

Increased dosage of both the above lipases improved the crumb structure and produced bread with better appearance and crust structure. At high dosage of the S3-4 produced enzyme, a decrease in bread volume appeared and the same tendency was observed for the S4-1 produced lipase at a lower dosage.
It is concluded that also these tested mutant lipases improved dough and bread stability and improved the crumb structure as does the wild-type lipase 3.

## INDICATIONS RELATING TO A DEPOSITED MICROORGANISM

### (PCT Rule 13bis)

| | |
|---|---|
| **A.** The indications made below relate to the microorganism referred to in the description | |
| on page __35__ , line __26__ . | |

**B. IDENTIFICATION OF DEPOSIT**            Further deposits are identified on an additional sheet [X]

Name of depositary institution       The National Collections of Industrial and Marine Bacteria Limited (NCIMB)

Address of depositary institution *(including postal code and country)*

23 St. Machar Drive, Aberdeen, Scotland, United Kingdom, AB2 1RY

| Date of deposit | Accession Number |
|---|---|
| 24 February 1997 | NCIMB 40863 |

**C. ADDITIONAL INDICATIONS** *(leave blank if not applicable)*   This information is continued on an additional sheet ☐

As regards the respective Patent Offices of the respective designated states, the applicants request that a sample of the deposited microorganisms only be made available to an expert nominated by the requester until the date on which the patent is granted or the date on which the application has been refused or withdrawn or is deemed to be withdrawn.

**D. DESIGNATED STATES FOR WHICH INDICATIONS ARE MADE** *(if the indications are not for all designated States)*




**E. SEPARATE FURNISHING OF INDICATIONS** *(leave blank if not applicable)*

The indications listed below will be submitted to the International Bureau later *(specify the general nature of the indications e.g., 'Accession Number of Deposit')*



| For receiving Office use only | For International Bureau use only |
|---|---|
| ☒ This sheet was received with the international application | ☐ This sheet was received by the International Bureau on: |
| Authorized officer   *Jette Cordes Paulsen*  Head Clerk | Authorized officer |

INDICATIONS RELATING TO DEPOSITED MICROORGANISMS
(PCT Rule 12*bis*)

**Additional sheet**

In addition to the microorganism indicated on page 80 of the description, the following microorganisms have been deposited with

 The National Collections of Industrial and Marine
 Bacteria Limited (NCIMB)
 23 St. Marchar Drive, Aberdeen, Scotland
 United Kingdom AB2 1RY

on the dates and under the accession numbers as stated below:

| Accession number | Date of deposit | Description Page No. | Description Line No. |
|---|---|---|---|
| NCIMB 40931 | 24 March 1998 | 45 | 1 |
| NCIMB 40932 | 24 March 1998 | 45 | 1 |
| NCIMB 40933 | 24 March 1998 | 45 | 1 |
| NCIMB 40934 | 24 March 1998 | 45 | 2 |
| NCIMB 40935 | 24 March 1998 | 45 | 3 |

For all of the above-identified deposited microorganisms, the following additional indications apply:

As regards the respective Patent Offices of the respective designated states, the applicants request that a sample of the deposited microorganisms stated above only be made available to an expert nominated by the requester until the date on which the patent is granted or the date on which the application has been refused or withdrawn or is deemed to be withdrawn.

SEQUENCE LISTING

(1) GENERAL, INFORMATION:

(i) APPLICANT:

(A) NAME: DANISCO A/S
(B) STREET: Langebrogade 1
(C) CITY: Copenhagen
(E) COUNTRY: Denmark
(F) POSTAL CODE (ZIP): 1001 K
(G) TELEPHONE: +45 32 66 22.00
(H) TELEFAX: +45 32 66 21 67

(ii) TITLE OF INVENTION: Cloning and use of Lipase 3 gene from Aspergillus tubigensis

(iii) NUMBER OF SEQUENCES: 9

(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SBQ ID NO: 1:

(i) SEQUENCE CHARACTERISTICS:

(A) LENGTH: 25 amino acids
(B) TYPE: amino acid
(C) STRANDEDNESS: single
(D) TOPOLOGY: linear

(ii) MOLECULE TYPE: peptide

(v) FRAGMENT TYPE: N-terminal

(vi) ORIGINAL SOURCE:

(A) ORGANISM: Aspergillus tubigensis

(xi) SEQUENCE DESCRIPTION: SEQ ID NO: 1:

```
Ser Val Ser Thr Ser Thr Leu Asp Glu Leu Gln Leu Phe Ala Gln Trp
1               5                   10                  15

Ser Ala Ala Ala Tyr Xaa Ser Asn Asn
                20              25
```

(2) INFORMATION FOR SEQ ID NO: 2:

(i) SEQUENCE CHARACTERISTICS:

(A) LENGTH: 7 amino acids
(B) TYPE: amino acid
(C) STRANDEDNESS: single
(D) TOPOLOGY: linear

(ii) MOLECULE TYPE: peptide
(v) FRAGMENT TYPE: internal
(vi) ORIGINAL SOURCE:

(A) ORGANISM: Aspergillus tubigensis

(xi) SEQUENCE DESCRIPTION: SEQ ID NO: 2:

```
                    Val His Thr Gly Phe Trp Lys
                     1               5
```

(2) INFORMATION FOR SEQ ID NO: 3:

(i) SEQUENCE CHARACTERISTICS:

(A) LENGTH: 14 amino acids
(B) TYPE: amino acid
(C) STRANDEDNESS: single
(D) TOPOLOGY: linear

(ii) MOLECULE TYPE: peptide
(v) FRAGMENT TYPE: internal
(vi) ORIGINAL SOURCE:

(A) ORGANISM: Aspergillus tubigensis

(xi) SEQUENCE DESCRIPTION: SEQ ID NO: 3:

```
            Ala Trp Glu Ser Ala Ala Asp Glu Leu Thr Ser Lys Ile Lys
             1               5                  10
```

(2) INFORMATION FOR SEQ ID NO: 4:

(i) SEQUENT CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Oligonucleotide"

(xi) SEQUENCE DESCRIPTION: SEQ ID NO: 4:
TTCCARAANC CNGTRTGNAC          20

(2) INFORMATION FOR SEQ ID NO: 5:

(i) SEQUENCE CHARACTERISTICS:

(A) LENGTH: 18 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Oligonucleotide"

(xi) SEQUENCE DESCRIPTION: SEQ ID NO: 5:
CARYTNTTYG CNCARTGG          18

(2) INFORMATION FOR SEQ ID NO: 6:

(i) SEQUENCE CHARACTERISTICS:

(A) LENGTH: 17 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Oligonucleotide"

(xi) SEQUENCE DESCRIPTION: SEQ ID NO: 6:
GCVGCHSM CCCKVGC          17

(2) INFORMATION FOR SEQ ID NO: 7:

(i) SEQUENCE CHARACTERISTICS:

(A) LENGTH: 317 base pairs
(B) TYPE: nucleic acid
(C) STRANDEDNESS: double
(D) TOPOLOGY: linear

(ii) MOLECOLE TYPE: other nucleic acid

(A) DESCRIPTION: /desc = "PCR fragment"

(xi) SEQUENCE DESCRIPTION: SEQ ID NO: 7:

```
CAGTTGTTCG CGCAATGGTC TGCCGCAGCT TATTGCTCGA ATAATATCGA CTCGAAAGAV  60

TCCAACTTGA CATGCACGGC CAACGCCTGT CCATCAGTCG AGGAGGCCAG TACCACGATG  120

CTGCTGGAGT TCGACCTGTA TGTCACTCAG ATCGCAGACA TAGAGCACAG CTAATTGAAC  180

AGGACGAACG ACTTTTGGAG GCACAGCCGG TTTCCTGGCC GCGGACAACA CCAACAAGCG  240

GCTCGTGGTC GCCTTCCGGG GAAGCAGCAC GATTGAGAAC TGGATTGCTA ATCYTGACTT  300

CATCCTGGRA GATAACG                                                  317
```

(2) INFORMATION FOR SEQ ID NO: 8:

(i) SEQUENCE CHARACTERISTICS:

(A) LENGTH: 1045 base pairs
(B) TYPE: nucleic acid
(C) STRANDEDNESS: double
(D) TOPOLOGY: linear

(ii) MOLECULE TYPE: DNA (genomic)
(vi) ORIGINAL SOURCE:

(A) ORGANISM: Aspergillus tubigensis

(ix) FEATURE:

(A) NAME/KEY: CDS
(B) LOCATION:join(1..82, 135..300, 347..683, 737..1045)

(ix) FEATURE:

(A) NAME/KEY: sig_peptide
(B) LOCATION:1..81

(ix) FEATURE:

(A) NAME/KEY: mat_peptide
(B) LOCATION:join (82, 135..300, 347..683, 737..1042)

(xi) SEQUENCE DESCRIPTION: SEQ ID NO: 8:

```
ATG TTC TCT GGA CGG TTT GGA GTG CTT TTG ACA GCG CTT GCT GCG CTG      48
Met Phe Ser Gly Arg Phe Gly Val Leu Leu Thr Ala Leu Ala Ala Leu
-27     -25                 -20                 -15


GGT GCT GCC GCG CCG GCA CCG CTT GCT GTG CGG A GTAGGTGTGC            92
Gly Ala Ala Ala Pro Ala Pro Leu Ala Val Arg
        -10                 -5


CCGATGTGAG ATGGTTGGAT AGCACTGATG AAGGGTGAAT AG   GT GTC TCG ACT    145
                                                 Ser Val Ser Thr
                                                 1


TCC ACG TTG GAT GAG TTG CAA TTG TTC GCG CAA TGG TCT GCC GCA GCT    193
Ser Thr Leu Asp Glu Leu Gln Leu Phe Ala Gln Trp Ser Ala Ala Ala
    5                 10                  15                  20


TAT TGC TCG AAT AAT ATC GAC TCG AAA GAC TCC AAC TTG ACA TGC ACG    241
Tyr Cys Ser Asn Asn Ile Asp Ser Lys Asp Ser Asn Leu Thr Cys Thr
                    25                  30                  35


GCC AAC GCC TGT CCA TCA GTC GAG GAG GCC AGT ACC ACG ATG CTG CTG    289
Ala Asn Ala Cys Pro Ser Val Glu Glu Ala Ser Thr Thr Met Leu Leu
            40                  45                  50
```

```
GAG TTC GAC CT   GTATGTCACT CAGATCGCAG ACATAGAGCA CAGCTAATTT      340
Glu Phe Asp Leu
            55


GAACAG G ACG AAC GAC TTT GGA GGC ACA GCC GGT TTC CTG GCC GCG GAC   389
         Thr Asn Asp Phe Gly Gly Thr Ala Gly Phe Leu Ala Ala Asp
                  60                  65                  70


AAC ACC AAC AAG CGG CTC GTG GTC GCC TTC CGG GGA AGC AGC ACG ATT   437
Asn Thr Asn Lys Arg Leu Val Val Ala Phe Arg Gly Ser Ser Thr Ile
                75                  80                  85


GAG AAC TGG ATT GCT AAT CTT GAC TTC ATC CTG GAA GAT AAC GAC GAC   485
Glu Asn Trp Ile Ala Asn Leu Asp Phe Ile Leu Glu Asp Asn Asp Asp
                90                  95                  100


CTC TGC ACC GGC TGC AAG GTC CAT ACT GGT TTC TGG AAG GCA TGG GAG   533
Leu Cys Thr Gly Cys Lys Val His Thr Gly Phe Trp Lys Ala Trp Glu
            105                 110                 115


TCC GCT GCC GAC GAA CTG ACG AGC AAG ATC AAG TCT GCG ATG AGC ACG   581
Ser Ala Ala Asp Glu Leu Thr Ser Lys Ile Lys Ser Ala Met Ser Thr
            120                 125                 130


TAT TCG GGC TAT ACC CTA TAC TTC ACC GGG CAC AGT TTG GGC GGC GCA   629
Tyr Ser Gly Tyr Thr Leu Tyr Phe Thr Gly His Ser Leu Gly Gly Ala
135                 140                 145                 150


TTG GCT ACG CTG GGA GCG ACA GTT CTG CGA AAT GAC GGA TAT AGC GTT   677
Leu Ala Thr Leu Gly Ala Thr Val Leu Arg Asn Asp Gly Tyr Ser Val
                155                 160                 165


GAG CTG GTGAGTCCTT CACAAAGGTG ATGGAGCGAC AATCGGGAAC AGACAGTCAA   733
Glu Leu


TAG TAC ACC TAT GGA TGT CCT CGA ATC GGA AAC TAT GCG CTG GCT GAG   781
    Tyr Thr Tyr Gly Cys Pro Arg Ile Gly Asn Tyr Ala Leu Ala Glu
        170                 175                 180


CAT ATC ACC AGT CAG GGA TCT GGG GCC AAC TTC CGT GTT ACA CAC TTG   829
His Ile Thr Ser Gln Gly Ser Gly Ala Asn Phe Arg Val Thr His Leu
    185                 190                 195
```

```
AAC GAC ATC GTC CCC CGG GTG CCA CCC ATG GAC TTT GGA TTC AGT CAG   877
Asn Asp Ile Val Pro Arg Val Pro Pro Met Asp Phe Gly Phe Ser Gln
200             205             210             215

CCA AGT CCG GAA TAC TGG ATC ACC AGT GGC AAT GGA GCC AGT GTC ACG   925
Pro Ser Pro Glu Tyr Trp Ile Thr Ser Gly Asn Gly Ala Ser Val Thr
                220             225             230

GCG TCG GAT ATC GAA GTC ATC GAG GGA ATC AAT TCA ACG GCG GGA AAT   973
Ala Ser Asp Ile Glu Val Ile Glu Gly Ile Asn Ser Thr Ala Gly Asn
        235             240             245

GCA GGC GAA GCA ACG GTG AGC GTT GTG GCT CAC TTG TGG TAC TTT TTT  1021
Ala Gly Glu Ala Thr Val Ser Val Val Ala His Leu Trp Tyr Phe Phe
        250             255             260

GCG ATT TCC GAG TGC CTG CTA TAA                                  1045
Ala Ile Ser Glu Cys Leu Leu  *
    265             270
```

(2) INFORMATION FOR SEQ ID NO: 9:

(i) SEQUENCE CHARACTERISTICS;

(A) LENGTH: 297 amino acids
(B) TYPE: amino acid
(D) TOPOLOGY: linear

(ii) MOLECULE TYPE: protein
(xi) SEQUENCE DESCRIPTION: SEQ ID NO: 9:

```
Met Phe Ser Gly Arg Phe Gly Val Leu Leu Thr Ala Leu Ala Ala Leu
-27     -25             -20             -15

Gly Ala Ala Ala Pro Ala Pro Leu Ala Val Arg Ser Val Ser Thr Ser
    -10             -5              1               5

Thr Leu Asp Glu Leu Gln Leu Phe Ala Gln Trp Ser Ala Ala Ala Tyr
                10              15              20

Cys Ser Asn Asn Ile Asp Ser Lys Asp Ser Asn Leu Thr Cys Thr Ala
            25              30              35

Asn Ala Cys Pro Ser Val Glu Glu Ala Ser Thr Thr Met Leu Leu Glu
        40              45              50
```

```
Phe Asp Leu Thr Asn Asp Phe Gly Gly Thr Ala Gly Phe Leu Ala Ala
    55                  60              65

Asp Asn Thr Asn Lys Arg Leu Val Val Ala Phe Arg Gly Ser Ser Thr
    70              75              80                      85

Ile Glu Asn Trp Ile Ala Asn Leu Asp Phe Ile Leu Glu Asp Asn Asp
                90              95                  100

Asp Leu Cys Thr Gly Cys Lys Val His Thr Gly Phe Trp Lys Ala Trp
            105             110                 115

Glu Ser Ala Ala Asp Glu Leu Thr Ser Lys Ile Lys Ser Ala Met Ser
        120             125                 130

Thr Tyr Ser Gly Tyr Thr Leu Tyr Phe Thr Gly His Ser Leu Gly Gly
    135                 140                 145

Ala Leu Ala Thr Leu Gly Ala Thr Val Leu Arg Asn Asp Gly Tyr Ser
    150             155                 160                 165

Val Glu Leu Tyr Thr Tyr Gly Cys Pro Arg Ile Gly Asn Tyr Ala Leu
            170                 175                 180

Ala Glu His Ile Thr Ser Gln Gly Ser Gly Ala Asn Phe Arg Val Thr
            185                 190                 195

His Leu Asn Asp Ile Val Pro Arg Val Pro Pro Met Asp Phe Gly Phe
            200                 205                 210

Ser Gln Pro Ser Pro Glu Tyr Trp Ile Thr Ser Gly Asn Gly Ala Ser
    215                 220                 225

Val Thr Ala Ser Asp Ile Glu Val Ile Glu Gly Ile Asn Ser Thr Ala
230                 235                 240                 245

Gly Asn Ala Gly Glu Ala Thr Val Ser Val Val Ala His Leu Trp Tyr
            250                 255                 260

Phe Phe Ala Ile Ser Glu Cys Leu Leu   *
            265                 270
```

## Claims

1. A method of preparing a baked product having improved pore homogeneity and reduced pore diameter comprising adding a polypeptide to a dough, wherein the polypeptide has lipase activity and is a triacylglycerol hydrolysing enzyme, which is capable of splitting off fatty acids having short, medium and long chain length, **characterised in**

**that** said polypeptide is capable of hydrolysing galactolipids that are normally present in the flour to the corresponding galactosyl monoglycerides and wherein said polypeptide is capable of hydrolysing at least 10% of the galactosyl diglycerides normally present in the flour dough to galactosyl monoglycerides and wherein said polypeptide retains at least 60% of its activity after 1 hour at 60°C in 100mM sodium acetate buffer at pH5.0.

2.  A method according to claim 1 wherein said polypeptide retains at least 80% activity after 4 days at 20°C and at a pH in the range of 3.5-8 including a pH in the range of 5-7.

3.  A method according to claim 1 or claim 2 wherein said polypeptide retains at least 80% of its activity after 1 hour at 50°C in 100mM sodium acetate buffer at pH5.0.

4.  A method according to any one of claims 1 to 3, wherein when said polypeptide is added to a bread dough in an amount of 5,000 lipase units per kg flour, the average pore diameter of the crumb of the bread made from the dough is reduced by at least 1.0%, relative to a bread which is made from a bread dough without addition of the lipase.

5.  A method according to any one of the preceding claims, wherein when said polypeptide is added to a bread dough in an amount of 5,000 lipase units per kg flour, the pore homogeneity of the crumb of the bread made from the dough is increased by at least 5%, relative to a bread which is made from a bread dough without addition of the lipase.

6.  A method according to any one of the preceding claims, wherein the dough and/or baked product further comprises an emulsifier.

7.  A method according to any one of the preceding claims, wherein the dough is a fat-free dough.

8.  A method according to any one of the preceding claims, wherein said polypeptide is capable of hydrolysing at least 25% of the galactosyl diglycerides normally present in the flour dough to galactosyl monoglycerides.

**Patentansprüche**

1.  Verfahren zur Herstellung eines gebackenen Produkts mit verbesserter Porenhomogenität und vermindertem Porendurchmesser, welches die Zugabe eines Polypeptids zu einem Teig umfaßt, wobei das Polypeptid Lipaseaktivität besitzt und ein Triacylglycerolhydrolysierendes Enzym ist, welches Fettsäuren mit kurzer, mittlerer und langer Kettenlänge abspalten kann, **dadurch gekennzeichnet, daß** das Polypeptid Galactolipide, die normalerweise in dem Mehl vorhanden sind, zu den entsprechenden Galactosylmonoglyceriden hydrolysieren kann und wobei das Polypeptid wenigstens 10 % der Galactosyldiglyceride, die normalerweise in dem Teig vorhanden sind, zu Galactosymonoglyceriden hydrolysieren kann und wobei das Polypeptid wenigstens 60 % seiner Aktivität nach einer Stunde bei 60 °C in 100 mM Natriumacetatpuffer bei einem pH-Wert von 5,0 beibehält.

2.  Verfahren nach Anspruch 1, wobei das Polypeptid wenigstens 80 % Aktivität nach vier Tagen bei 20 °C und bei einem pH-Wert im Bereich von 3,5 bis 8, einschließlich einem pH-Wert im Bereich von 5 bis 7, beibehält.

3.  Verfahren nach Anspruch 1 oder Anspruch 2, wobei das Polypeptid wenigstens 80 % seiner Aktivität nach einer Stunde bei 50 °C in 100 mM Natriumacetatpuffer bei einem pH-Wert von 5,0 beibehält.

4.  Verfahren nach einem der Ansprüche 1 bis 3, wobei das Polypeptid zu einem Brotteig in einer Menge von 5000 Lipaseeinheiten pro Kilogramm Mehl hinzugefügt wird und der mittlere Porendurchmesser der Krume des aus dem Teig hergestellten Brotes um wenigstens 10 % gegenüber einem Brot, das aus einem Brotteig ohne Zugabe der Lipase hergestellt ist, reduziert wird.

5.  Verfahren nach einem vorangegangenen Ansprüche, wobei das Polypeptid zu einem Brotteig in einer Menge von 5000 Lipaseeinheiten pro Kilogramm Mehl hinzugefügt wird und die Porenhomogenität der Krume des aus dem Teig hergestellten Brotes um wenigstens 5 % gegenüber einem Brot, das aus einem Brotteig ohne eine Zugabe der Lipase hergestellt ist, erhöht wird.

6.  Verfahren nach einem der vorangegangenen Ansprüche, wobei der Teig und/oder das gebackene Produkt weiterhin ein Emulgiermittel umfassen.

**7.** Verfahren nach einem der vorangegangenen Ansprüche, wobei der Teig ein fettfreier Teig ist.

**8.** Verfahren nach einem der vorangegangenen Ansprüche, wobei das Polypeptid wenigstens 25 % der Galactosyldiglyceride, die normalerweise in dem Mehlteig vorhanden sind, zu Galactosymonoglyceriden hydrolysieren kann.

## Revendications

**1.** Procédé pour la préparation d'un produit cuit au four ayant une homogénéité des pores améliorée et un diamètre des pores réduit, comprenant l'addition d'un polypeptide à une pâte, dans lequel le polypeptide a une activité de lipase et est une enzyme hydrolysant les triacylglycérols, qui est capable de scinder les acides gras ayant des longueurs de chaîne courtes, moyennes et grandes, **caractérisé en ce que** ledit polypeptide est capable d'hydrolyser les galactolipides qui sont normalement présents dans la farine en les galactosyl-monoglycérides correspondants et ledit polypeptide est capable d'hydrolyser au moins 10 % des galactosyl-diglycérides normalement présents dans la pâte de farine en galactosyl-monoglycérides, et ledit polypeptide conserve au moins 60 % de son activité après 1 heure à 60°C dans du tampon à l'acétate de sodium 100 mM à pH 5,0.

**2.** Procédé suivant la revendication 1, dans lequel ledit polypeptide conserve au moins 80 % de son activité après 4 jours à 20°C et à un pH compris dans la plage de 3,5 à 8, y compris un pH de la plage de 5 à 7.

**3.** Procédé suivant la revendication 1 ou la revendication 2, dans lequel ledit polypeptide conserve au moins 80 % de son activité après 1 heure à 50°C dans du tampon à l'acétate de sodium 100 mM à pH 5,0.

**4.** Procédé suivant l'une quelconque des revendications 1 à 3, dans lequel, lorsque ledit polypeptide est ajouté à une pâte à pain en une quantité de 5000 unités de lipase par kg de farine, le diamètre moyen des pores de la mie du pain préparé à partir de la pâte est réduit d'au moins 10 %, par rapport à un pain qui est préparé à partir d'une pâte à pain sans addition de la lipase.

**5.** Procédé suivant l'une quelconque des revendications précédentes, dans lequel, lorsque ledit polypeptide est ajouté à une pâte à pain en une quantité de 5000 unités de lipase par kg de farine, l'homogénéité des pores de la mie du pain préparé à partir de la pâte est augmentée d'au moins 5 %, par rapport à un pain qui est préparé à partir d'une pâte à pain sans addition de la lipase.

**6.** Procédé suivant l'une quelconque des revendications précédentes, dans lequel la pâte et/ou le produit cuit au four comprennent en outre un émulsionnant.

**7.** Procédé suivant l'une quelconque des revendications précédentes, dans lequel la pâte est une pâte sans matière grasse.

**8.** Procédé suivant l'une quelconque des revendications précédentes, dans lequel ledit polypeptide est capable d'hydrolyser au moins 25 % des galactosyl-diglycérides normalement présents dans la pâte de farine en galactosyl-monoglycérides.

Kpn I (9000)
Pst I (8850)
Sma I (8700)
Nco I (8400)
Bst 11071 (7435)
intron1
intron2
Sac II (7043)
Lip3
intron3
Sma I (6581)
Nco I (6567)
Eco RV (6492)
Pvu II (6153)
Sma I (6140)
Pst I (5300)
Kpn I (5200)
Bst 11071 (5000)

Pst I (11)
Sma I (15)
BamH I (19)
Spe I (25)
Xba I (31)
Not I (38)
Sac II (50)
Sac I (59)
Pvu II (277)

pLIP4
9396 bp

Pvu II (2790)
Kpn I (2915)
Xho I (2926)
Sal I (2932)
Cla I (2942)
Hind III (2947)
Eco RV (2955)
Sma I (3800)

**Fig. 1**

**Fig. 2**

1834 bp

BEST TRANSFORMANT

Fig. 3

CONTROL - UNTRANSFORMED STRAIN

**Fig. 4**